# EUROPÄISCHE PATENTSCHRIFT

(11) **EP 3 705 152 B1**
(45) Veröffentlichungstag und Bekanntmachung des Hinweises auf die Patenterteilung: **26.10.2022**
(21) Anmeldenummer: 20000088.3
(22) Anmeldetag: 02.03.2020
(51) Int. Cl.: A61M 16/01, A61M 16/12, A61M 16/10, A61M 16/20, A61M 16/00, A61M 16/08

(54) **BEATMUNGSGERÄT MIT MISCHERKAMMER UND MISCHERKAMMER FÜR EIN BEATMUNGSGERÄT**
VENTILATOR WITH MIXER CHAMBER AND MIXER CHAMBER FOR A VENTILATOR
APPAREIL RESPIRATOIRE POURVU DE CHAMBRE DE MÉLANGEUR ET CHAMBRE DE MÉLANGEUR POUR UN APPAREIL RESPIRATOIRE

(30) Priorität: 07.03.2019 DE 102019001657
(43) Veröffentlichungstag der Anmeldung: 09.09.2020
(73) Patentinhaber: Löwenstein Medical Technology S.A., 2557 Luxembourg (LU)
(72) Erfinder: Mulfinger, Jonas, 61267 Neu-Anspach (DE)
(74) Vertreter: Marx, Thomas

(56) Entgegenhaltungen:
- EP-A1- 2 425 869
- WO-A1-02/28460
- WO-A1-2016/140980
- WO-A1-2017/055995
- DE-A1-102014 109 394
- DE-B3-102006 055 779
- US-A1- 2012 006 326

## Beschreibung

Die Erfindung betrifft ein Beatmungsgerät, welches für die Unterstützung oder Aufrechterhaltung der Atemfunktion eines Patienten vorgesehen ist und ein Elektrogebläse und/oder ein Ventil als Atemgasquelle aufweist. Bei dem Patienten kann es sich hierbei um einen Menschen oder um ein luftatmendes Tier, beispielsweise um ein Säugetier, handeln. Die Erfindung betrifft insbesondere ein Beatmungsgerät, bei welchem der abgegebenen Atemluft mindestens ein Zusatzgas beigemischt ist. Die Erfindung betrifft weiter ein Beatmungsgerät, welches als Narkosegerät ausgebildet ist.

Ein wesentlicher sicherheitskritischer Aspekt von Beatmungsgeräten ist die Gewährleistung der Zufuhr von Atemgas bei einem Ausfall der Atemgasquelle. Eine Herausforderung bei Beatmungsgeräten ist die bedarfsgerechte Mischung der applizierten Gase, sowie eine effektive Schalldämmung. DE 102006055779 B3 offenbart eine Gasmischvorrichtung für Beatmungsgeräte mit einem Vorratsbehälter, in den Druckluft, Sauerstoff und Gas aus der Umgebung eingeleitet werden können, so dass dem Patienten ein Gasgemisch zugeführt werden kann.

Die folgenden Dokumente aus dem Stand der Technik offenbaren Beatmungsgeräte mit Mischerkammern und entsprechenden Ventilanordnungen, DE 10 2006 055779, EP 2 425 869, US 2012/006326, DE 10 2014 109394, WO 2017/05599, WO 02/28460 und WO 2016/140980.

Die Aufgabe der Erfindung besteht darin, ein gegenüber dem Stand der Technik verbessertes Beatmungsgerät zur Verfügung zu stellen, das eine kompakte Bauweise aufweist und trotzdem sicher ausgebildet ist.

Diese Aufgabe wird erfindungsgemäß durch das vorgeschlagene Beatmungsgerät nach Anspruch 1 gelöst.

Das offenbarte Beatmungsgerät weist eine elektronische Steuervorrichtung auf, sowie eine pneumatische Hauptleitung in der pneumatisch verbunden angeordnet sind; eine Atemgasquelle, die als Elektrogebläse mit einem Ansaugeingang und einem Gebläseausgang ausgebildet ist, zumindest ein Ventil, eine Mischkammer, eine Gasdosierungseinheit und eine Zuleitung wobei die Gasdosierungseinheit eingerichtet ist Außenluft und/oder Sauerstoff und/oder Narkosegas in die Mischkammer zu leiten und dass die Atemgasquelle eingerichtet ist Atemgas zu der Zuleitung zu fördern und dass die Mischkammer eingerichtet ist Atemgas bereitzustellen, wobei die Zuleitung zur Versorgung des Patienten mit Atemgas eingerichtet ist.

Das offenbarte Ventil ist unter anderem dazu eingerichtet, einen Atemgasstrom zum Patienten zumindest zeitweise zu reduzieren, wobei das Atemgas Außenluft und/oder Sauerstoff und/oder Narkosegas aufweist, wobei das Ventil als Teil der Mischkammer ausgebildet ist oder in einem gemeinsamen Gehäuse der Mischkammer angeordnet ist, wobei das Ventil in der pneumatischen Hauptleitung in Durchflussrichtung nach dem Gebläseausgang angeordnet ist und in Durchflussrichtung vor der Gasdosierungseinheit, wobei die Gasdosierungseinheit in Durchflussrichtung vor der Zuleitung angeordnet ist.

Die Mischkammer ist eingerichtet, Atemgas bereitzustellen indem Außenluft und/oder Sauerstoff und/oder Narkosegas gemischt werden.

Die Atemgasquelle ist in der pneumatischen Hauptleitung und als Elektrogebläse ausgebildet und ein Gebläseausgang ist pneumatisch mit dem Ventil und das Ventil mit der Mischkammer und die Mischkammer sowohl mit einer Gasdosierungseinheit als auch mit einer Zuleitung pneumatisch verbunden, wobei der Eingang des Elektrogebläse zur Bereitstellung von Außenluft eingerichtet ist, wobei die Gasdosierungseinheit für eine einstellbare pneumatische Einspeisung von einem Sauerstoff enthaltenden Zusatzgas zu oder anstelle der geförderten Außenluft in die Mischkammer ausgebildet ist, wobei die Zuleitung zur Versorgung des Patienten mit einem Atemgas bestehend aus der geförderten Außenluft oder einem Gasgemisch aus der Außenluft und dem Zusatzgas oder aus dem Zusatzgas allein eingerichtet ist, wobei das Ventil eingerichtet ist, einen Strom von Außenluft in die Mischkammer zumindest zeitweise zu reduzieren oder zu unterbrechen.

In der pneumatischen Hauptleitung ist in Durchflussrichtung der Gebläseausgang mit dem Ventil der Mischkammer und die Mischkammer sowohl mit einer Gasdosierungseinheit als auch mit einer Zuleitung pneumatisch verbunden, wobei der Ansaugeingang zur Förderung von Außenluft eingerichtet ist, wobei die Gasdosierungseinheit für eine einstellbare pneumatische Einspeisung von einem Sauerstoff enthaltenden Zusatzgas zu oder anstelle der geförderten Außenluft in die Mischkammer ausgebildet ist, wobei die Zuleitung zur Versorgung des Patienten mit einem Atemgas bestehend aus der geförderten Außenluft oder einem Gasgemisch aus der Außenluft und dem Zusatzgas oder aus dem Zusatzgas allein eingerichtet ist, wobei mit der Steuervorrichtung der Zusatzgasanteil, der Beatmungsdruck und ein Beatmungsfluss des Atemgases einstellbar ist und wobei die Steuervorrichtung erfindungsgemäß und zusätzlich zur Absperrung des Ventils unter gleichzeitiger Öffnung der Gasdosierungseinheit sowie die Gasdosierungseinheit selbst zur Bereitstellung von Atemgas bei Ausfall des Elektrogebläses und/oder der Stromversorgung und/oder bei Ausfall des Prozessors und/oder bei Absturz der Software ausgebildet ist.

Die Mischkammer weist einen Anschluss für die Gasdosierungseinheit, einen Anschluss für die Zuleitung und einen Anschluss für die Atemgasquelle auf.

Die Mischkammer weist zumindest einen Anschluss zur Verbindung mit einer Komponente auf, wobei der Anschluss einen lösbaren Verschluss zur schnellen Montage der Komponente aufweist. Der lösbare Verschluss ist beispielsweise ein Bajonett-Verschluss und dient der schnellen Montage, Positionierung und Abdichtung. Die Mischkammer weist Mittel zur Reduzierung des Luftschalls und Mittel zur Vermischung der Gase auf. Zudem weist die Mischkammer bevorzugt Mittel auf, die eine Sauerstoffleckage bei Sauerstoffbeatmung verhindern und Mittel, die eine Rückatmung des Patienten in das Beatmungsgerät verhindern.

Das Ventil weist einen Zulauf und einen Auslauf in einem Ventilgehäuse auf und ist über den Auslauf mit dem Ansaugeingang oder über den Zulauf mit dem Gebläseausgang pneumatisch verbunden, wobei das Elektrogebläse und das Ventil mit der Steuervorrichtung in mindestens einem gemeinsamen Regelkreis elektronisch geregelt sind und die Steuervorrichtung selbst zumindest teilweise über einen Algorithmus in Form von Software mit einem Prozessor elektronisch regelbar und/oder automatisch geregelt ist, wobei in oder an der pneumatischen Hauptleitung und/oder in weiteren pneumatischen Zweig- und/oder Nebenleitungen und/oder Rückführungen funktionelle Baugruppen und optional Mess- und/oder Regeleinstrumente pneumatisch geschaltet sind, wobei die funktionellen Baugruppen mittels der Steuervorrichtung elektronisch geregelt und die Mess- und/oder Regeleinstrumente ebenfalls optionale Baugruppen der Steuervorrichtung sind.

Das Ventil ist als Magnetventil und/oder als Proportionalventil ausgebildet.

Das Ventil ist bevorzugt als Magnetventil ausgebildet, mit einem in einem Ventilgehäuse fixierten Elektromagneten und mit einem magnetisch bewegbaren Ventilkolben, wobei der Ventilkolben einen Dichtteller mit einer Dichtung aufweist, wobei der Dichtteller auf einen Zulauf wirkt und wobei der Ventilkolben mit dem Dichtteller durch eine Feder gegen den Zulauf gepresst wird, sodass ein Gasfluss von dem oder zu dem Elektrogebläse unterbunden ist.

Der Elektromagnet ist, im geschlossenen Zustand des Magnetventils, stromlos.

Wenn der Elektromagnet bestromt wird, wirkt eine einstellbare oder vorbestimmte Magnetkraft auf den magnetisch bewegbaren Ventilkolben ein, wobei der magnetisch bewegbare Ventilkolben die Feder vorgebbar weit komprimiert, wobei in einem geöffneten Zustand die Magnetkraft, die auf den magnetisch bewegbaren Ventilkolben einwirkt, größer ist als die Federkraft.

Die Steuervorrichtung weist zumindest einen Prozessor (oder Computer) auf oder mehrere Prozessoren um zumindest das Elektrogebläse, das Ventil und Mess- und/oder Regeleinstrumente zu steuern.

Die Steuervorrichtung ist erfindungsgemäß zur automatischen Absperrung des Ventils bei Ausfall des Elektrogebläses und/oder bei Ausfall der Steuervorrichtung ausgebildet.

Das beanspruchte Beatmungsgerät weist eine elektronische Steuervorrichtung sowie ein Elektrogebläse mit einem Ansaugeingang und einem Gebläseausgang auf. Das Beatmungsgerät ist zur Förderung von Atemgas oder Narkosegas mit dem Elektrogebläse durch eine pneumatische Hauptleitung zu einem Patienten, das heißt, für eine Gasversorgung des Patienten, ausgebildet. Das Beatmungsgerät weist weiter ein Ventil auf, wobei das Ventil selbst ein Ventilgehäuse mit einem Zulauf und ein Auslauf aufweist. Das Ventilgehäuse als Baugruppe ist bis auf den Zulauf und den Ablauf gasdicht. Das Elektrogebläse und das Ventil sind innerhalb der pneumatischen Hauptleitung über den Auslauf des Ventils mit dem Ansaugeingang oder über den Zulauf des Ventils mit dem Gebläseausgang des Elektrogebläses pneumatisch miteinander verbunden. Bei dem beanspruchten Beatmungsgerät ist somit eine Durchflussrichtung eines Gases oder einer Gasmischung allgemein vom Elektrogebläse zu dem Patienten festgelegt, welche somit unabhängig davon ist, ob das Ventil vor dem Ansaugeingang oder nach dem Gebläseausgang pneumatisch geschaltet ist.

Das Ventil ist elektronisch regelbar. Hierbei sind das Elektrogebläse als auch das Ventil mit der Steuervorrichtung in mindestens einem gemeinsamen Regelkreis elektronisch geregelt, wobei die Steuervorrichtung selbst zumindest teilweise über einen Algorithmus in Form von Software mit einem Prozessor elektronisch regelbar und/oder automatisch geregelt ist. Hierzu weist das Beatmungsgerät eine Stromversorgung auf, welche für die Versorgung aller elektrisch betriebenen und elektrisch und/oder elektronisch gesteuerten Gerätebauteile des vorgeschlagenen Beatmungsgerätes eingerichtet ist. Die Stromversorgung ist beispielsweise durch einen Anschluss an ein elektrisches Versorgungsnetz realisiert.

Bei dem vorgeschlagenen Beatmungsgerät ist die Steuervorrichtung zur Regulierung der Gasversorgung des Patienten bezüglich des Beatmungsdruckes und des Beatmungsflusses über die Steuervorrichtung ausgebildet. Die Regulierung der Gasversorgung des Patienten ist hierbei über eine Leistungsregelung des Elektrogebläses bewerkstelligbar. Zusätzlich oder alternativ ist die Gasversorgung des Patienten über eine Regulierung des Ventils mit der Steuervorrichtung eingerichtet, wobei das Ventil als Stetigventil, insbesondere als Proportionalventil, ausgebildet ist.

Weiter weist das beanspruchte Beatmungsgerät optional Flussund/oder Drucksensoren auf, welche in oder an der pneumatischen Hauptleitung und/oder in weiteren pneumatischen Zweig- und/oder Nebenleitungen und/oder Rückführungen des Beatmungsgerätes pneumatisch geschaltet sind. Die Fluss- und Drucksensoren sind durch die Steuervorrichtung elektronisch ansteuerbar. Die Flussund/oder Drucksensoren sind somit optionale Baugruppen der Steuervorrichtung und daher mit der Steuervorrichtung elektronisch geschaltet. Über die mit den Fluss- und/oder Drucksensoren gemessenen Druck- oder Flusswerte des durch die pneumatische Hauptleitung strömenden Gases oder Gasmischung, insbesondere Narkose- oder Atemgas, sind mit der elektronischen Steuervorrichtung unter Regelung des Ventils und/oder unter Leistungsregelung des Elektrogebläses gewünschte oder festgelegte Druck- und/oder Flusswerte dieses Gases oder der Gasmischung einstellbar.

Im Vergleich zu herkömmlichen Beatmungsgeräten, welche ein Elektrogebläse aufweisen, sind der Beatmungsdruck und der Beatmungsfluss mit der Steuervorrichtung des vorgeschlagenen Beatmungsgerätes wesentlich präziser einstellbar. Somit weist das vorgeschlagene Beatmungsgerät eine höhere Betriebssicherheit auf. Weiter ist das Ventil des vorgeschlagenen Beatmungsgerätes zur weiteren Verbesserung der Betriebssicherheit zusätzlich als Sperrventil ausgebildet.

In oder an der pneumatischen Hauptleitung und/oder in weiteren pneumatischen Zweig- und/oder Nebenleitungen und/oder Rückführungen sind je nach vorgesehenem Verwendungszweck des vorgeschlagenen Beatmungsgerätes weitere funktionelle Baugruppen und/oder neben den Fluss- und/oder Drucksensoren weitere solche Mess- und/oder Regeleinstrumente pneumatisch geschaltet, wobei die Mess- und/oder Regeleinstrumente ebenfalls optionale Baugruppen der Steuervorrichtung sind. Solche funktionelle Baugruppen sind beispielsweise eine Gasdosierungseinheit, eine Mischkammer und eine Zuleitung. Die Gasdosierungseinheit ist für pneumatische Einspeisungen von Gasen in die Mischkammer ausgebildet. Die Mischkammer ist zur Vermischung der eingespeisten Gase eingerichtet. Die Zuleitung selbst ist über ein Interface direkt zu den Atmungsorganen des Patienten geführt, wobei das Interface beispielswiese durch eine Beatmungsmaske oder einen Tubus gegeben ist. Die funktionellen Baugruppen sind mit der Steuervorrichtung elektronisch ebenfalls geregelt. In oder an der pneumatischen Hauptleitung und/oder in weiteren pneumatischen Zweig- und/oder Nebenleitungen und/oder Rückführungen sind hierbei optional je nach Reqelunqs- und/oder Verwendungszweck auch mehr als nur ein Ventil geschaltet.

Das vorgeschlagene Beatmungsgerät ist in einer typischen Ausführungsform für einen Betrieb bei einem Betriebsdruck von maximal 1 mbar bis 2 mbar bei geöffnetem Elektrogebläse bei einem maximalen Gasfluss 180 l/min bis 200 l/min ausgebildet, wobei der Betriebsdruck als auch der Gasfluss auch auf andere Werte einstellbar ist. Bei geschlossenem Elektrogebläse ist ein Druckaufbau bis zu einem Grenzdruck dadurch limitiert, dass sich bei Überschreitung des Grenzdruckes das Ventil öffnet. In einer typischen Ausführungsform des beanspruchten Beatmungsgerätes ist der Grenzdruck auf 100 mbar festgelegt, jedoch ist eine konstruktive Auslegung des Ventils auch für andere Grenzdrücke realisierbar.

Nachfolgend wird auf vorteilhafte Weiterbildungen des vorgeschlagenen Beatmungsgerätes hinsichtlich von Sicherheitsaspekten und bezüglich ihres Verwendungszweckes eingegangen.

Erfindungsgemäß ist die Steuervorrichtung des vorgeschlagenen Beatmungsgerätes zur automatischen Absperrung des Ventils bei Ausfall des Elektrogebläses und/oder bei Ausfall des Prozessors und/oder bei Absturz der Software ausgebildet. Somit ist bei Ausfall des Prozessors und/oder bei Absturz der Software eine Totraumatmung des Patienten durch das Elektrogebläse und ein Rückfluss von Gasen durch den Gebläseausgang in das Elektrogebläse besonders vorteilhaft verhindert, wenn das Ventil vor dem Gebläseausgang pneumatisch geschaltet ist. Ist ein Ventil vor dem Ansaugeingang pneumatisch geschaltet, so ist ein Eindringen von Gasen oder Außenluft durch diese Öffnung des Elektrogebläses verhindert.

In einer zweiten Weiterbildung ist das vorgeschlagene Beatmungsgerät als Narkosegerät ausgebildet. In dem vorgeschlagenen Beatmungsgerät ist in Durchflussrichtung eine Gasdosierungseinheit mit einer Mischkammer pneumatisch verbunden. Die Mischkammer ist wiederum mit dem Ansaugeingang des Elektrogebläses und der Gebläseausgang mit einer Zuleitung pneumatisch verbunden. Somit bilden in dieser pneumatischen Schaltungsfolge bezüglich der Durchflussrichtung die Gasdosierungseinheit, die Mischkammer, das Elektrogebläse und die Zuleitung die pneumatische Hauptleitung aus, wobei das Ventil vor dem Ansaugeingang oder nach dem Gebläseausgang oder jeweils ein Ventil vor dem Ansaugeingang und nach dem Gebläseausgang in der pneumatischen Hauptleitung pneumatisch geschaltet ist oder sind.

Ist das oder ein Ventil vor dem vor dem Ansaugeingang angeordnet, so ist der Zulauf mit der Mischkammer und der Auslauf mit dem Ansaugeingang pneumatisch verbunden. Ist das oder ein Ventil nach dem Gebläseausgang angeordnet, so ist der Zulauf mit dem Gebläseausgang und der Auslauf mit der Zuleitung pneumatisch verbunden.

Hierbei sind die Gasdosierungseinheit für pneumatische Einspeisungen von Gasen und von mindestens einem Anästhetikum in die Mischkammer, die Mischkammer zur Mischung eines Narkosegases aus den eingespeisten Gasen und der Ansaugeingang zur Förderung von Narkosegas ausgebildet. Die Zuleitung ist zur Versorgung des Patienten mit Narkosegas eingerichtet, wobei das Narkosegas Sauerstoff und mindestens ein Anästhetikum enthält. Optional ist in bei dem beanspruchten Beatmungsgerät in dieser Ausführungsform an der pneumatischen Hauptleitung zusätzlich ein Rückatmungssystem pneumatisch angeschlossen, welches besonders vorteilhaft eine Rückführung von unverbrauchtem Anästhetikum zu dem Patienten unter Vermeidung einer Emission in die Umgebung ermöglicht.

Mit der Steuervorrichtung sind die pneumatischen Einspeisungen von Gasen und von mindestens einem Anästhetikum durch die Gasdosierungseinheit jeweils unabhängig voneinander bezüglich ihrer Stoffmengenanteile im Narkosegas einstellbar, das heißt, die Einspeisung eines jeden Gases und Anästhetikums in die Mischkammer ist unabhängig von den anderen Gasen mit der Gasdosierungseinheit bezüglich Druck- und/oder Volumen- und/oder Fluss einstellbar. Dies schließt die Auswahl oder Beschränkung auf ein Gas oder auf einzelne Gase und/oder Anästhetikum mit ein, welche für die Einspeisung in die Mischkammer vorgesehen sind. Mit der Steuervorrichtung ist ebenfalls der Beatmungsdruck und der Beatmungsfluss des in der Mischkammer angemischten Narkosegases einstellbar und damit regelbar. Erfindungsgemäß ist die Steuervorrichtung zur automatischen Absperrung des Ventils und zusätzlich zur automatischen Absperrung der Gasdosierungseinheit bei Ausfall des Elektrogebläses und/oder der Stromversorgung und/oder bei Ausfall des Prozessors und/oder bei Absturz der Software ausgebildet.

In einer dritten Weiterbildung, welche komplementär zu der zweiten Weiterbildungsform ist, ist das vorgeschlagene Beatmungsgerät ausschließlich für eine Versorgung eines Patienten mit Atemgas ausgelegt. In dieser Ausführungsform ist in Durchflussrichtung der Gebläseausgang mit dem Ventil pneumatisch verbunden. Der Auslauf des Ventils ist wiederum mit einer Mischkammer pneumatisch verbunden, wobei an die Mischkammer sowohl eine Gasdosierungseinheit als auch eine Zuleitung pneumatisch angeschlossen sind. Auch bei dieser Ausführungsform des vorgeschlagenen Beatmungsgerätes ist die Zuleitung wie anfangs beschrieben zu den Atmungsorganen des Patienten geführt. Somit bilden bei dem vorgeschlagenen Beatmungsgerät in dieser Ausführungsform in pneumatischer Schaltungsreihenfolge bezüglich der Durchflussrichtung der Ansaugeingang, der Gebläseausgang mit dem Ventil, der Auslauf des Ventils mit der Mischkammer und die Mischkammer sowohl mit einer Gasdosierungseinheit als auch mit der Zuleitung die pneumatische Hauptleitung aus.

Hierbei sind jeweils der Ansaugeingang zur Förderung von Außenluft und die Gasdosierungseinheit für eine einstellbare pneumatische Einspeisung von einem Sauerstoff enthaltenden Zusatzgas zu oder anstelle der geförderten Außenluft in die Mischkammer ausgebildet. Die ist Zuleitung zur Versorgung des Patienten mit einem Atemgas bestehend aus der geförderten Außenluft oder einem Gasgemisch aus der Außenluft und dem Zusatzgas oder aus dem Zusatzgas allein eingerichtet. Mit der Steuervorrichtung sind der Zusatzgasanteil, der Beatmungsdruck und ein Beatmungsfluss des Atemgases einstellbar.

Hierbei ist die Steuervorrichtung zur automatischen Absperrung des Ventils und zusätzlich zur automatischen Absperrung des Ventils unter gleichzeitiger Öffnung der Gasdosierungseinheit bei Ausfall des Elektrogebläses und/oder der Stromversorgung und/oder bei Ausfall des Prozessors und/oder bei Absturz der Software ausgebildet.

Bei Ausfall des Elektrogebläses und/oder der Stromversorgung und/oder bei Ausfall des Prozessors und/oder bei Absturz der Software ist die Gasdosierungseinheit zur vollautomatischen und/oder teilunterstützen Beatmung des Patienten eingerichtet. Hierzu weist die Gasdosierungseinheit eine von der Steuervorrichtung unabhängige elektronische Ersatzsteuervorrichtung und eine von der Stromversorgung unabhängige Ersatzstromversorgung auf. Sowohl die Ersatzsteuervorrichtung als auch die Ersatzstromversorgung sind bei Eintritt mindestens eines der vorbenannten Ausfälle zur automatischen Einschaltung und Versorgung mit elektrischem Strom der Gasdosierungseinheit eingerichtet. Hierbei sind das Elektrogebläse, das Ventil als auch die Gasdosierungseinheit mit der Ersatzsteuervorrichtung in mindestens einem gemeinsamen Regelkreis elektronisch geregelt, wobei die Ersatzsteuervorrichtung ebenfalls selbst zumindest teilweise über einen Algorithmus in Form von Software mit einem Prozessor elektronisch regelbar und/oder automatisch geregelt ist. Die Ersatzstromversorgung ist beispielsweise durch Akkumulatoren ausgebildet.

Nachfolgend werden vorteilhafte Varianten des Ventils des beanspruchten Beatmungsgerätes näher beschrieben.

In einer ersten bevorzugten Variante ist das Ventil direktgesteuert. Hierfür weist das Ventil einen in dem Ventilgehäuse fixierten Hubelektromagneten mit einem magnetisch anziehbaren Ventilkolben auf, wobei der Ventilkolben in dem Hubelektromagneten lineargelagert ist. Der Ventilkolben ist aus einem magnetisch anziehbaren Material, beispielsweise aus Eisen oder einer Eisenlegierung gefertigt und/oder selbst als ein Permanentmagnet ausgebildet. Der Ventilkolben ist auf einer geometrischen Longitudinalachse in dem Hubelektromagneten mittels eines elektrischen Stromes verschiebbar, wobei eine Rückstellfeder in dem Ventilgehäuse mit dem Ventilkolben endseitig verspannt ist. Die Rückstellfeder weist eine Rückstellkraft parallel zu der Longitudinalachse auf. Die Rückstellfeder ist somit in Richtung der Longitudinalachse expandierbar, wobei mit dem elektrisch eingeschalteten Hubelektromagneten eine durch den Ventilkolben ausgeübte Kraft größer als die Rückstellkraft einstellbar ist.

Durch Stromregelung am Hubmagneten ist das Ventil somit optional als Proportionalventil betreibbar. Dies gestattet besonders vorteilhaft eine präzise Einstellung und/oder Nachregelung von festgelegten Druck- und/oder Flusswerten des Narkosegases oder des Atemgases.

Auf dem Ventilkolben ist vorderseitig und vertikal ein Dichtteller angebracht. Der Dichtteller ist auf der gemeinsamen geometrischen Longitudinalachse mit dem Ventilkolben elektrisch verschiebbar. Der Zulauf ist als Ventilsitz ausgebildet, wobei hierfür der Dichtteller gegenüber einer elastischen Flanschdichtung auf dem Zulauf angeordnet ist.

Im geschlossenen Zustand des Ventils, wobei der Hubelektromagnet stromlos ist, ist der Dichtteller auf die Flanschdichtung durch die Rückstellkraft der Rückstellfeder auf die Flanschdichtung aufgepresst. Somit ist in diesem Schaltungszustand der Zulauf durch die Flanschdichtung abgedichtet. Dem Dichtteller ist zusätzlich ein mechanisches Spiel, beispielsweise über ein Kugelgelenk, mit drei Freiheitsgraden gegeben. Nur so ist eine sichere Abdichtung, im stromlosen Zustand des Elektrohubmagneten, durch Ausgleich einer fertigungsbedingten Winkeltoleranz des Ventils gewährleistet. Das mechanische Spiel ist beispielsweise durch eine auf dem der Dichtteller aufgebrachte Kugelpfanne realisiert, wobei die Kugelpfanne auf einer Kugel auf dem Ventilkolben eingerastet ist. Optional ist auf dem Dichtteller eine umlaufende Dichtkante aufgebracht, wobei die Dichtkante im geschlossenen Zustand des Ventils zusammen mit dem Dichtteller auf die Flanschdichtung aufgepresst und somit die Abdichtung besonders vorteilhaft verstärkt ist. Die Flanschdichtung ist aus einem elastischen Material, beispielsweise Silikon oder Butadien-Kautschuk, gefertigt. Der Dichtteller dagegen ist aus einem harten oder elastischen Material, beispielsweise aus Metall oder ABS oder Silikon oder Kautschuk, gefertigt.

Vorzugsweise ist das beanspruchte Beatmungsgerät konstruktiv so ausgebildet, dass in Durchflussrichtung zu dem Patienten eine Laminarströmung des Gasflusses in der pneumatischen Leitung realisiert ist. Je besser eine Laminarströmung ausgebildet ist, dass heißt, je weniger Strömungsturbulenzen gegeben sind, desto präziser ist sowohl der Gasdruck als auch der Gasfluss messbar und einstellbar.

Zu diesem Zweck, dass heißt zur Verhinderung von Strömungsturbulenzen, weisen der Zulauf als auch der Auslauf jeweils einen gleichen Strömungsquerschnitt bezüglich Flächengröße auf. Beim Betrieb des beanspruchten Beatmungsgerätes und geöffneten Zustand des Ventils ist weiter ein Gasfluss sowohl auf der dem Zufluss zugewandten Seite des Dichttellers als auch dahinter konstruktionsbedingt ermöglicht, indem hierfür um den Hubelektromagneten und innerhalb des Ventilgehäuses Freiräume für einströmendes Gas gegeben sind. Beim Betrieb des beanspruchten Beatmungsgerätes und geöffneten Zustand des Ventils entspricht hierfür weiter das mathematische Produkt aus dem Umfang des Dichttellers und dem Abstand zwischen dem Dichtteller und dem durch die Flanschdichtung ausgebildeten Rand des Zulaufes dem Strömungsquerschnitt mit einer Abweichung von nicht mehr als 20 %. Bei einem optionalen Betrieb des Ventils als Proportionalventil ist der Abstand zwischen dem Dichtteller und dem Rand des Zulaufes einstellbar.

Vorzugsweise sind der Zulauf, das Ventilgehäuse, der Hubelektromagnet, der Ventilkolben, der Dichtteller zusammen mit der optionalen Dichtkante und die Flanschdichtung bezüglich der Longitudinalachse rotationssymmetrisch gefertigt. Damit ist der Ausgleich der fertigungsbedingten Winkeltoleranz des Ventils optimiert. Dies ist auch für einen Erhalt der Laminarströmung vorteilhafter.

Erfindungsgemäß ist das Ventil zur Absperrung des Elektrogebläses bei Ausfall der Stromversorgung ausgebildet.

Nachfolgend sind Weiterentwicklungen des vorgeschlagenen Beatmungsgerätes bezüglich der Mischkammer beschrieben, wobei das Beatmungsgerät ausschließlich für eine Versorgung eines Patienten mit Atemgas ausgelegt ist. Hierbei ist das Ventil des beanspruchten Beatmungsgerätes optional in seiner zuvor beschriebenen ersten und/oder zweiten bevorzugten Variante ausgeführt.

In einer solchen ersten Weiterentwicklung bezüglich der Mischkammer ist die Mischkammer als Ventilgehäuse ausgebildet und somit das Ventil in die Mischkammer integriert. Hierbei sind der Zulauf des Ventils als pneumatischer Zulauf zu der Mischkammer ausgebildet und der Auslauf in eine Innenkammer der Mischkammer pneumatisch geführt. Die Innenkammer ist sowohl über einen Anschluss mit der Gasdosierungseinheit als auch über einen weiteren Anschluss mit der Zuleitung pneumatisch verbunden. Auf diese Weise ist besonders vorteilhaft eine kompakte Bauweise des beanspruchten Beatmungsgerätes realisiert.

Vorzugsweise ist der Strömungsquerschnitt des Zulaufes des Ventils bezüglich Flächengröße als auch Geometrie identisch mit dem jeweiligen

Strömungsquerschnitt sowohl des Anschlusses für die Gasdosierungseinheit als auch des Anschlusses für die Zuleitung. Hierdurch ist besonders vorteilhaft ein Erhalt der Laminarströmung durch die Mischkammer ist realisiert.

Versuche selbst mit schallgedämpften Elektrogebläsen haben gezeigt, dass durch Übertragung von Vibrationen und vor allem durch den Gasfluss auf funktionelle Baugruppen, welche in der pneumatischen Hauptleitung auf Seite des Gebläseausganges angeordnet sind, die funktionellen Baugruppen eine höhere Schallentwicklung als durch das Elektrogebläse selbst aufweisen. Insbesondere ist ein weiter wesentlicher Beitrag zur unerwünschten Schallentwicklung durch den Betrieb einer Gasdosierungseinheit gegeben.

Daher weist optional die Innenkammer zusätzlich ein Labyrinth zur Dämpfung des durch den Betrieb des Elektrogebläses und der Gasdosierungseinheit erzeugten Schalls auf. Es hat sich überraschend gezeigt, dass mit Lochblenden anstatt eines Labyrinthes eine weitaus geringere Schalldämpfung realisierbar ist.

In einer zweiten Weiterentwicklung des vorgeschlagenen Beatmungsgerätes bezüglich der Mischkammer weist oder weisen zusätzlich in dem Labyrinth der Fluss der geförderten Außenluft und/oder der Fluss des eingespeisten Zusatzgases und/oder der Fluss des Atemgases jeweils mindestens einmal eine Umlenkung auf. Hierdurch ist besonders vorteilhaft eine Rückreflexion der durch das Elektrogebläse erzeugten Schallwellen bewirkt.

Alternativ oder zusätzlich ist oder sind in dem Labyrinth der Fluss der geförderten Außenluft und/oder der Fluss des eingespeisten Zusatzgases und/oder der Fluss des Atemgases jeweils mindestens einmal eine Änderung des durchflossenen Querschnittes vertikal zu der Durchflussrichtung aufweist oder aufweisen. Diese zweite Weiterentwicklung entspricht einer Verbesserung der konstruktiven Merkmale des Labyrinthes bezüglich einer Schalldämmung.

In einer dritten Weiterentwicklung des vorgeschlagenen Beatmungsgerätes bezüglich der Mischkammer weist die Mischkammer vor dem Anschluss der Gasdosierungseinheit einen Umlenkkeil mit einer Keilspitze auf, wobei der Fluss des eingespeisten Sauerstoffes um die Keilspitze herumgeführt ist. Hierbei ist der Umlenkkeil hohlförmig ausgebildet oder weist eine Füllung aus Schaumstoff auf. Durch den Umlenkkeil ist hierbei sowohl eine Zusammenführung des Flusses der geförderten Außenluft mit dem Fluss des eingespeisten Zusatzgases zu dem Fluss des Atemgases gegeben, wodurch eine bessere Durchmischung erzielt ist. Vorzugsweise ist die Verengung am Stumpf des Umlenkkeiles zur Innenwand der Innenkammer so hinreichend groß gewählt, dass hierbei eine Verdichtung, das heißt Druckerhöhung, des Gasflusses nicht mehr messbar gegeben ist. Ist der Umlenkkeil hohlförmig ausgebildet, so ist besonders vorteilhaft ein Brechen der durch das Elektrogebläse und die Gasdosierungseinheit erzeugten Schallwellen und damit deren Dämpfung gegeben.

Die Dämpfung der Schallwellen ist dadurch zusätzlich verbessert, dass der Umlenkkeil mit Schaumstoff gefüllt ist. Eine weitere Verbesserung der Schalldämpfung ist dann gegeben, wenn das beanspruchte Beatmungsgerät zusätzlich die Merkmale der zweiten Weiterentwicklung des vorgeschlagenen Beatmungsgerätes bezüglich der Mischkammer aufweist.

In einer vierten Weiterentwicklung des vorgeschlagenen Beatmungsgerätes bezüglich der Mischkammer ist in dem Labyrinth eine Oberfläche gegeben, welche zumindest teilweise mit makroporösem Schaumstoff ausgekleidet ist. Hierdurch und insbesondere zusätzlich in Kombination mit mindestens einem der Merkmale der ersten bis dritten Weiterentwicklung des vorgeschlagenen Beatmungsgerätes bezüglich der Mischkammer ist eine weitere Optimierung der Schalldämpfung erzielbar.

Die Oberfläche in dem Labyrinth weist bei dieser vierten Weiterentwicklung des vorgeschlagenen Beatmungsgerätes bezüglich der Mischkammer alternativ oder zusätzlich zumindest in Teilbereichen antimikrobielle Eigenschaften auf, wobei der Schaumstoff selbst optional antimikrobielle Eigenschaften aufweist. Hierzu ist beispielsweise die Mischkammer aus einem Kunststoff, beispielsweise Polystyrol (PS) oder ABS, gefertigt welchem beispielsweise Silberzeolithe oder Silberpartikel beigefügt sind. Analog hierzu sind dem Schaumstoff beispielsweise biozide Silber-Additiva beigemischt. Alternativ weisen die Oberfläche des Labyrinthes und/oder der Schaumstoff eine antimikrobielle Oberflächenbeschichtung auf, welche beispielsweise Silber und/oder andere biozide Metalle und/oder chemische Verbindungen davon und/oder biozide quarternäre Ammonium- und/oder Phosphoniumsalze enthält. Somit ist besonders vorteilhaft bei einer eventuellen Rückatmung eines aktuell behandelten Patienten durch die Zuleitung die Gefahr einer Kontamination der Mischkammer mit Mikroben gebannt, welche für eine Infektion für nachfolgend behandelte Patienten ansonsten bestünde.

Die Mischkammer des vorgeschlagenen Beatmungsgerätes, welches ausschließlich für eine Versorgung eines Patienten mit Atemgas ausgelegt ist, ist beispielsweise aus einem Baustück und einem Gegenstück zu einem Bauteil zusammengesetzt. Das Baustück und das Gegenstück sind unabhängig voneinander jeweils aus einem gleichen oder verschiedenen inelastischen Material gefertigt. Vorzugsweise sind das Baustück und das Gegenstück kostengünstig aus einem Thermoplast, beispielsweise ABS, mittels Spritzguss gefertigt. Das Baustück und das Gegenstück weisen jeweils Verbindungskanten auf, welche formschlüssig zueinander sind. Die Mischkammer ist auch dadurch gekennzeichnet, dass die Mischkammer ein Mischkammergehäuse aufweist, welches einen Anschluss für die Gasdosierungseinheit, einen Anschluss für die Zuleitung und einen Anschluss für die Atemgasquelle aufweist. Die Mischkammer ist auch dadurch gekennzeichnet, dass die Mischkammer ein aus einem Baustück und einem Gegenstück formund kraftschlüssig gefertigtes Bauteil, beispielsweise ein Mischkammergehäuse aufweist, wobei das Baustück und das Gegenstück jeweils zueinander formschlüssige Verbindungskanten aufweisen, wobei bei dem Baustück mindestens in eine Verbindungskante (612) eine Nut parallel zu der Länge der Verbindungskante und eine in die Nut vertikal mündende und die betreffende Verbindungskante durchbrechende Quernut eingelassen ist, wobei eine sowohl in der Nut als auch in der Quernut verlaufende einstückige elastische und komprimierbare Flachdichtung eingebracht ist, wobei die Flachdichtung formschlüssig sowohl zu der Nut als auch zu der Quernut ist, wobei der Flachdichtung eine Dichtungshöhe größer als die Tiefe der Nut als auch die Tiefe der Quernut und maximal entsprechend sowohl der doppelten Tiefe der Nut als auch der doppelten Tiefe der Quernut gegeben ist und wobei die Flachdichtung über die Quernut durch die Verbindungkante um nicht mehr als der doppelten Tiefe der Quernut entsprechend hinausragend gestaltet ist.

Die Verbindungskante oder Verbindungskanten des Baustückes weisen jeweils eine durchgängige Nut über die gesamte Länge der Verbindungskante auf. In jede Nut mit einer Nuttiefe ist hierbei eine Flachdichtung formschlüssig zur Nut eingebracht, beispielsweise durch Einspritzen eines Kunststoffes. Die Flachdichtung ist aus einem elastischen Material gefertigt, beispielsweise Silikon oder Butadien-Kautschuk. Die Flachdichtung weist eine Dichtungshöhe auf, welche größer als die Nuttiefe ist.

Das Baustück und das Gegenstück sind mit der innenliegenden Flachdichtung form- und kraftschlüssig miteinander verbunden. Die kraftschlüssige Verbindung ist beispielsweise durch eine Verschraubung realisiert. Hierbei ist eine Abdichtung der Mischkammer durch eine Anpressung der jeweiligen Flachdichtung in die jeweilige Nut des Baustückes und auf die Verbindungskante des Gegegenstückes realisiert.

Bei diesem aus dem Baustück und dem Gegenstück gefertigten Bauteil ist es zusätzlich konstruktiv gegeben, dass die Öffnungen für die Zuleitung und den Zulauf durch jeweilige Verbindungskanten des Baustückes und des Gegenstückes geteilt sind. Nach der bisherigen Lehre der Fertigungstechnik sind solche Konstruktionen allgemein zu vermeiden, da diese nach dem Stand der Technik unvermeidlich zu Undichtigkeiten an den betreffenden Öffnungen führen, wie beispielswiese an der Öffnung für die Zuleitung und für den Zulauf. Auch ab einer gewissen Länge der Verbindungskante sind Undichtigkeiten nicht vermeidbar.

Dieses Problem kann jedoch dadurch gelöst werden, dass das Baustück und das Gegenstück jeweils zueinander formschlüssige Verbindungskanten aufweisen, wobei bei dem Baustück mindestens in eine Verbindungskante eine Nut parallel zur Länge der Verbindungskante und eine in die Nut vertikal mündende und die betreffende Verbindungskante durchbrechende Quernut eingelassen ist. Die Quernut ist an derjenigen Stelle der betreffenden Verbindungskante eingelassen, an welcher ansonsten potentiell oder tatsächlich eine Undichtigkeit nach erfolgtem Kraftschluss auftritt.

Weiter ist für eine Abdichtung eine sowohl in der Nut als auch in der Quernut verlaufende einstückige elastische und komprimierbare Flachdichtung eingebracht, wobei die Flachdichtung formschlüssig sowohl zu der Nut als auch zu der Quernut ist, dass heißt, das die Flachdichtung sich über die gesamte Nut und über die gesamte Quernut erstreckend gestaltet ist. Der Flachdichtung ist eine Dichtungshöhe größer als die Tiefe der Nut als auch der Quernut und maximal entsprechend sowohl der doppelten Tiefe der Nut als auch der doppelten Tiefe der Quernut gegeben. Die Flachdichtung ist über die Quernut durch die Verbindungkante um nicht mehr als der doppelten Tiefe der Quernut entsprechend hinausragend gestaltet. Durch Kompression der Flachdichtung in der Quernut durch Form- und Kraftschluss zu dem Bauteil ist an der betreffenden Stelle der Verbindungskante die Abdichtung verstärkt.

Diese fertigungstechnische Problemlösung ist auf ein beliebiges aus einer beliebigen Anzahl an Baustücken und Gegenstücken formund kraftschlüssig gefertigtes Bauteil übertragbar. Die Fertigung der Bauteile, der Gegenstücke und der Flachdichtungen ist hierbei keinesfalls auf Kunststoffe beschränkt. Beispielsweise sind die Baustücke und die Gegenstücke aus Stahl und die Flachdichtungen aus Kupfer gefertigt.

Nachfolgend wird das vorgeschlagene Beatmungsgerät anhand einer Zeichnung näher erläutert. Hierin zeigt:
- Fig. 1: Das beanspruchte Beatmungsgerätes 1, welches als Narkosegerät ausgebildet ist;
- Fig. 2: Das beanspruchte Beatmungsgerätes 1, welches zur Beatmung eines Patienten mit Atemgas ausgebildet ist;
- Fig. 3: Aufsicht auf ein Ventil 3 des Beatmungsgerätes 1;
- Fig. 4: Querschnitt durch das Ventil 3 im geschlossenen Zustand;
- Fig. 5: Querschnitt durch das Magnetventil 3 im geöffneten Zustand;
- Fig. 6: Teilgeschnittene Seitenansicht des Beatmungsgerätes 1, welches zur Beatmung eines Patienten mit Atemgas ausgebildet ist und eine Mischkammer 6 aufweist, in welcher das Magnetventil 3 integriert ist;
- Fig. 7: Schrägansicht der Mischkammer 6, in welcher das Ventil 3 integriert ist, links in geschlossener Form, rechts im Querschnitt;
- Fig. 8: Querschnitt der Seitenansicht der Mischkammer 6, in welcher das Ventil 3 integriert ist, unter Verdeutlichung von Umlenkungen r;
- Fig. 9: Geschnittene Schrägansicht der Mischkammer 6, in welcher das Ventil 3 integriert ist, unter Verdeutlichung Änderungen eines jeweils durchflossenen Querschnittes Q₁, Q₂, Q₃, Q₄ und Q₅;
- Fig. 10: Seitenansicht eines form- und kraftschlüssig gefertigten Bauteils 601, beispielsweise eines Mischkammergehäuses 601, aus einem Baustück 610 und einem Gegenstück 611;
- Fig. 11: Aufsicht auf eine Verbindungskante 612 des Baustückes 610 unter Verdeutlichung der Verbindungskante 612 mit einer Nut 614, einer Quernut 615 und einer Flachdichtung 616.

In den nachfolgenden Ausführungsbeispielen weist das Beatmungsgerät 1 eine elektronische Steuervorrichtung sowie ein Elektrogebläse 2 mit einem Ansaugeingang 21 und einem Gebläseausgang 22 auf. Das Beatmungsgerät ist zur Förderung von Atemgas oder Narkosegas mit dem Elektrogebläse 2 zu den Atmungsorganen eines Patienten, das heißt, für eine Gasversorgung des Patienten ausgebildet.

Das Beatmungsgerät weist weiter ein Ventil 3 auf, wobei das Ventil 3 selbst eine Ventilgehäuse 301 mit einem Zulauf 302 und ein Auslauf 303 aufweist. In den nachfolgenden Ausführungsbeispielen sind das Elektrogebläse 2 als auch das Ventil 3 mit der Steuervorrichtung elektronisch geregelt. Das Elektrogebläse 2 ist mit dem Ventil 3 pneumatisch in einer pneumatischen Hauptleitung 4 verbunden. Bei dem beanspruchten Beatmungsgerät 1 ist allgemein eine Durchflussrichtung d eines Gases oder einer Gasmischung vom Elektrogebläse 2 zu dem Patienten festgelegt.

In den nachfolgenden Ausführungsbeispielen ist die Steuervorrichtung selbst über einen Algorithmus in Form von Software mit einem Prozessor elektronisch regelbar und/oder automatisch geregelt. Hierzu weist das Beatmungsgerät 1 eine Stromversorgung auf, welche für die Versorgung aller elektrisch betriebenen und elektrisch und/oder elektronisch gesteuerten Gerätebauteile des vorgeschlagenen Beatmungsgerätes eingerichtet ist. Die Stromversorgung ist durch einen Anschluss an ein elektrisches Versorgungsnetz realisiert.

In den nachfolgenden Ausführungsbeispielen des Beatmungsgerätes ist die Steuervorrichtung zur Regulierung der Gasversorgung des Patienten bezüglich des Beatmungsdruckes und des Beatmungsflusses über die Steuervorrichtung ausgebildet. Die Regulierung der Gasversorgung des Patienten ist hierbei über eine Leistungsregelung des Elektrogebläses bewerkstelligt. Weiter weist das beanspruchte Beatmungsgerät Fluss- und/oder Drucksensoren auf, welche in oder an der pneumatischen Hauptleitung des Beatmungsgerätes pneumatisch geschaltet sind. Die Fluss- und Drucksensoren sind durch die Steuervorrichtung elektronisch ansteuerbar und mit der Steuervorrichtung elektronisch geschaltet.

Das Beatmungsgerät ist erfindungsgemäß ein CPAP oder APAP oder Bilevel oder ein Heimbeatmungsgerät oder ein klinisches Beatmungsgerät oder ein Narkosebeatmunqsqerät. Das Ventil ist erfindungsgemäß als ein pneumatisch oder elektronisch gesteuertes Magnetventil oder Rückschlagventil oder Proportionalventil ausgeführt.

In den nachfolgenden Ausführungsbeispielen kann das Ventil 3 auch direktgesteuert ausgebildet sein. Hierzu ist die Steuervorrichtung des vorgeschlagenen Beatmungsgerätes zur automatischen Absperrung des Ventils 3 bei Ausfall des Elektrogebläses 2 und/oder bei Ausfall des Prozessors und/oder bei Absturz der Software ausgebildet.

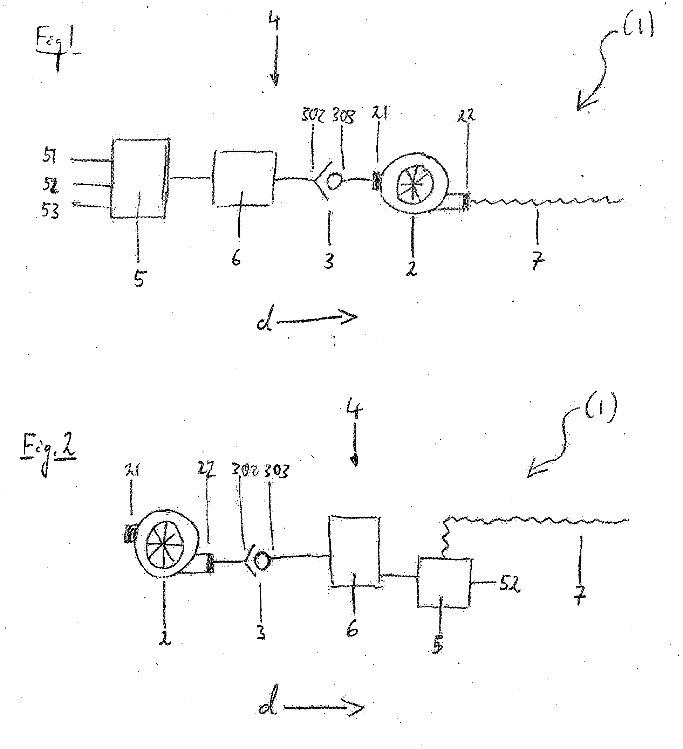

In den Fig. 1 bis Fig. 9 sind die Steuervorrichtung, der Prozessor, die Stromversorgung, die Fluss- und Drucksensoren sowie der Patient und seine Atmungsorgane nicht gezeigt.

In den nachfolgenden Ausführungsbeispielen weist das Beatmungsgerät eine Gasdosierungseinheit 5, ein Mischkammer 6 und eine Zuleitung 7 auf. Die Zuleitung 7 selbst ist direkt über einen Schlauch und/oder Tubus zu den Atmungsorganen des Patienten geführt. Der Tubus ist ebenfalls In den Fig. 1 bis Fig. 9 nicht gezeigt. In der Fig. 1, der Fig. 2 und der Fig. 6 sind das jeweils unterschiedliche pneumatische Schaltbild der pneumatischen Hauptleitung 4 repräsentativ für diese beiden unterschiedlichen Ausführungsbeispiele dargestellt.

Die Fig. 1 zeigt das beanspruchte Beatmungsgerät 1, welches beispielsweise zur Versorgung eines Patienten mit einem Narkosegas, ausgebildet ist. In Durchflussrichtung d, verdeutlicht durch einen Pfeil, ist die Gasdosierungseinheit 5 ist mit der Mischkammer 6 pneumatisch verbunden. Die Mischkammer 6 ist wiederum mit dem Zulauf 302 und der Auslauf 303 des Ventils 3 mit dem Ansaugeingang 21 des Elektrogebläses 2 pneumatisch geschaltet. Der Gebläseausgang 22 ist mit der Zuleitung 7 pneumatisch verbunden. Somit bilden in dieser pneumatischen Schaltungsfolge in einer Reihe und bezüglich der Durchflussrichtung d die Gasdosierungseinheit 5, die Mischkammer 6, das Ventil 3, das Elektrogebläse 2 und die Zuleitung die pneumatische Hauptleitung 4 aus. Die Gasdosierungseinheit 5 ist für pneumatische Einspeisungen von Außenluft, Sauerstoff und Lachgas in die Mischkammer 6, die Mischkammer 6 zur Mischung eines Narkosegases aus diesen eingespeisten Gasen und der Ansaugeingang 21 zur Förderung von Narkosegas ausgebildet. Das Narkosegas ist somit aus Außenluft, Sauerstoff und Lachgas zusammengesetzt. Die Gasdosierungseinheit 5 weist somit einen Einspeisungseingang für Außenluft 51, einen Einspeisungseingang für Sauerstoff 52 und einen Einspeisungseingang für Narkosegas (Lachgas) 53 auf, wobei Sauerstoff und Lachgas aus Druckgasflaschen zugeführt werden. Die Druckgasflaschen sind in der Fig. 1 nicht gezeigt. Die Zuleitung 7 ist zur Versorgung des Patienten mit

Atemgas z.B. Narkosegas eingerichtet. Mit der Steuervorrichtung (8) sind pneumatischen Einspeisungen dieser Gase bezüglich ihrer Stoffmengenanteile jeweils unabhängig voneinander, der Beatmungsdruck und ein Beatmungsfluss des Narkosegases einstellbar. Die Steuervorrichtung (8) ist zusätzlich zur automatischen Absperrung der Gasdosierungseinheit bei Ausfall des Elektrogebläses 2 und/oder der Stromversorgung und/oder bei Ausfall des Prozessors und/oder bei Absturz der Software ausgebildet. An der pneumatischen Hauptleitung 4 ist eine pneumatische Rückführung angeschlossen, wobei die pneumatische Rückführung zur Rückführung von Narkosegas in die pneumatische Hauptleitung 4 ausgebildet ist. Die pneumatische Rückführung ist in der Fig. 1 nicht gezeigt.

Die Fig. 2 zeigt das beanspruchte Beatmungsgerät 1, welches als zweites Ausführungsbeispiel zur Beatmung eines Patienten mit Atemgas ausgebildet ist. Hier sind dagegen in Durchflussrichtung d, wieder verdeutlicht durch einen Pfeil, in der pneumatischen Hauptleitung 4 der Gebläseausgang 22 mit dem Ventil 3, das Ventil 3 mit der Mischkammer 6 und die Mischkammer 6 sowohl mit der Gasdosierungseinheit 5 als auch mit der Zuleitung 7 pneumatisch verbunden. Der Ansaugeingang 21 ist zur Förderung von Außenluft eingerichtet. Die Gasdosierungseinheit 5 ist für eine einstellbare pneumatische Einspeisung von Sauerstoff als Zusatzgas zu oder anstelle der geförderten Außenluft in die Mischkammer 6 ausgebildet. Somit weist die Gasdosierungseinheit 5 hier nur einen Einspeisungseingang für Sauerstoff 52 auf, wobei der Sauerstoff wieder über eine Druckgasflasche zugeführt ist. Die Druckgasflasche ist in der Fig. 2 nicht gezeigt. Die Zuleitung 7 ist zur Versorgung des Patienten mit dem Atemgas bestehend aus der geförderten Außenluft oder einem Gasgemisch aus der Außenluft und Sauerstoff oder reinem Sauerstoff eingerichtet. Mit der Steuervorrichtung (8) ist der Sauerstoffanteil, der Beatmungsdruck und ein Beatmungsfluss des Atemgases einstellbar. In diesem zweiten Ausführungsbeispiel ist die Steuervorrichtung (8) zusätzlich zur automatischen Absperrung des Ventils 3 unter gleichzeitiger Öffnung der Gasdosierungseinheit 5 sowie die Gasdosierungseinheit 5 selbst zur vollautomatischen und/oder teilunterstützen Beatmung des Patienten bei Ausfall des Elektrogebläses 2 und/oder der Stromversorgung und/oder bei Ausfall des Prozessors und/oder bei Absturz der Software ausgebildet. Hierzu weist die Gasdosierungseinheit 5 beispielsweise eine separate unabhängige Steuervorrichtung (8) und separate Stromversorgung in Form von Akkumulatoren auf. Die separate unabhängige Steuervorrichtung (8) und die Akkumulatoren sind in der Fig. 2 nicht gezeigt.

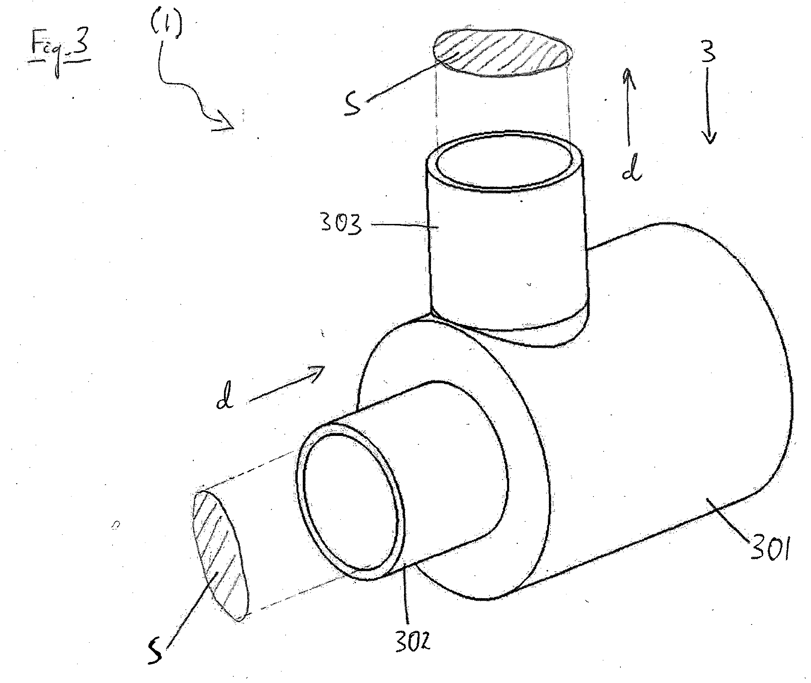

Die Fig. 3 zeigt eine Aufsicht auf ein Ventil 3 des beanspruchten Beatmungsgerätes 1, welches beispielsweise für die ersten beiden Ausführungsbeispiele als Ventil oder Magnetventil oder Proportionalventil ausgeführt ist. Das Ventilgehäuse 301 als Baugruppe ist bis auf den Zulauf 302 und den Auslauf 303 gasdicht. Die durch den Zulauf 302 und aus dem Auslauf 303 verlaufende Durchflussrichtung d ist wieder zeichnerisch durch einen Pfeil verdeutlicht. Sowohl der Zulauf 302 als auch der Auslauf 303 weisen einen sowohl bezüglich Flächengröße als auch Geometrie gleichen Strömungsquerschnitt S auf. Auf diese Weise ist ein Erhalt einer Laminarströmung unter Verhinderung von Strömungsturbulenzen gewährleistet. In der Fig. 3 sind alle weiteren Bauteile des Ventils 3 nicht gezeigt; diese Bauteile sind von dem Ventilgehäuse 301 umschlossen.

Der Öffnungsquerschnitt des Ventils beträgt zumindest 180 mm² und bevorzugt rund 230mm².

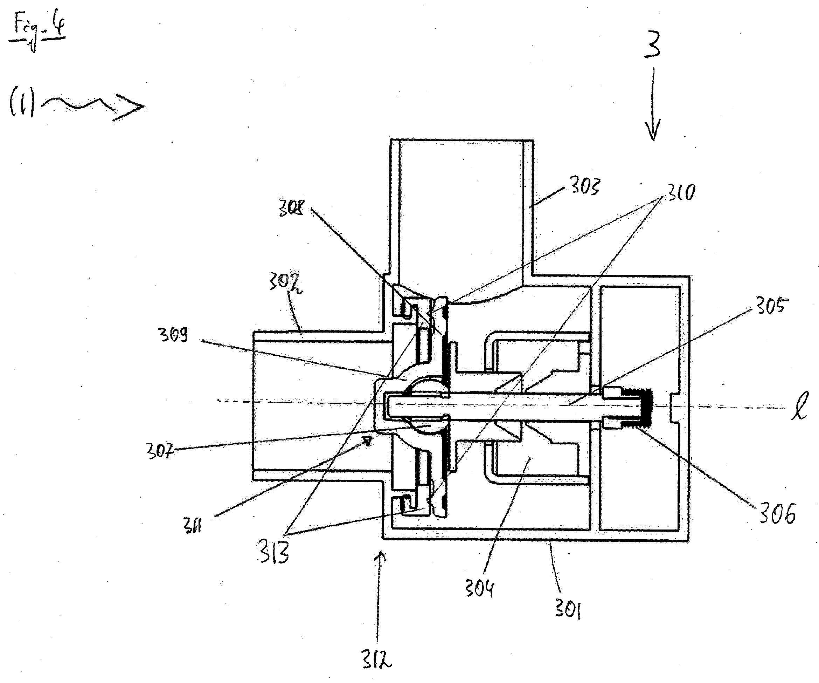

Die Fig. 4 zeigt einen Querschnitt durch das Ventil 3 des Beatmungsgerätes 1 aus Fig. 3 im geschlossenen Zustand. Hier ist das Ventil ein elektrisch geschaltetes Ventil mit einem Magneten. Im geschlossenen Zustand ist das Magnetventil 3 stromlos geschaltet. Das Magnetventil 3 weist einen in dem Ventilgehäuse 301 fixierten Hubelektromagneten 304 mit einem magnetisch anziehbaren Ventilkolben 305 auf, wobei der Ventilkolben 305 in dem Hubelektromagneten 304 lineargelagert ist. Der Ventilkolben 305 ist hier aus Nickelstahl gefertigt. Der Ventilkolben 305 ist auf einer geometrischen Longitudinalachse l in dem Hubelektromagneten 304 mittels eines elektrischen Stromes verschiebbar, wobei eine Rückstellfeder 306 in dem Ventilgehäuse 301 mit dem Ventilkolben 305 endseitig verspannt ist. Die Rückstellfeder 306 weist eine Rückstellkraft parallel zu der Longitudinalachse l auf. Die Rückstellfeder 306 ist somit in Richtung der Longitudinalachse l expandierbar, wobei mit dem elektrisch eingeschalteten Hubelektromagneten 304 eine durch den Ventilkolben 305 ausgeübte Kraft größer als die Rückstellkraft einstellbar ist. Auf dem Ventilkolben 305 ist vorderseitig und vertikal über eine auf dem Ventilkolben 305 aufgeschraubte Kugel 307 ein Dichtteller 308 eingerastet, wofür der Dichtteller 308 eine Kugelpfanne 309 aufweist. Der Dichtteller 308 weist weiter eine umlaufende Dichtkante 310 auf, wobei hier der Dichtteller 308 zusammen mit der Kugelpfanne 309 und der Dichtkante 310 einstückig mittels Spritzguss aus ABS gefertigt ist. Somit bilden die Kugel 307 und die die Kugelpfanne 309 ein Kugelgelenk 311 aus. Der Dichtteller 308 ist auf der Longitudinalachse l mit dem Ventilkolben 305 unter Öffnung des Magnetventils 3 elektrisch verschiebbar. Der Zulauf 302 ist somit als Ventilsitz 312 ausgebildet, wobei der Dichtteller 308 gegenüber einer elastischen Flanschdichtung 313 auf dem Zulauf 302 angeordnet ist. Die Flanschdichtung 313 ist hier aus Silikon gefertigt. Im geschlossenen Zustand des Magnetventils 3, wobei der Hubelektromagnet 304 stromlos ist, ist durch die Rückstellfeder 306 der Dichtteller 308 zusammen mit der Dichtkante 310 auf die Flanschdichtung 313 aufgepresst. Dem Dichtteller 308 ist über das Kugelgelenk 311 zusätzlich ein mechanisches Spiel mit drei Freiheitsgraden gegeben. Somit ist in diesem geschlossenen und stromlosen Zustand der Zulauf 302 durch die Flanschdichtung 313 auch dadurch besonders sicher abgedichtet, dass durch das mechanische Spiel zusätzlich ein Ausgleich einer fertigungsbedingten Winkeltoleranz des Magnetventils 3 gewährleistet ist. Der Zulauf 302, das Ventilgehäuse 301, der Hubelektromagnet 304, der Ventilkolben 305, der Dichtteller 308 zusammen mit der Dichtkante 310 und die Flanschdichtung 313 sind bezüglich der Longitudinalachse l rotationssymmetrisch gefertigt. Damit ist der Ausgleich der fertigungsbedingten Winkeltoleranz des Magnetventils 3 optimiert.

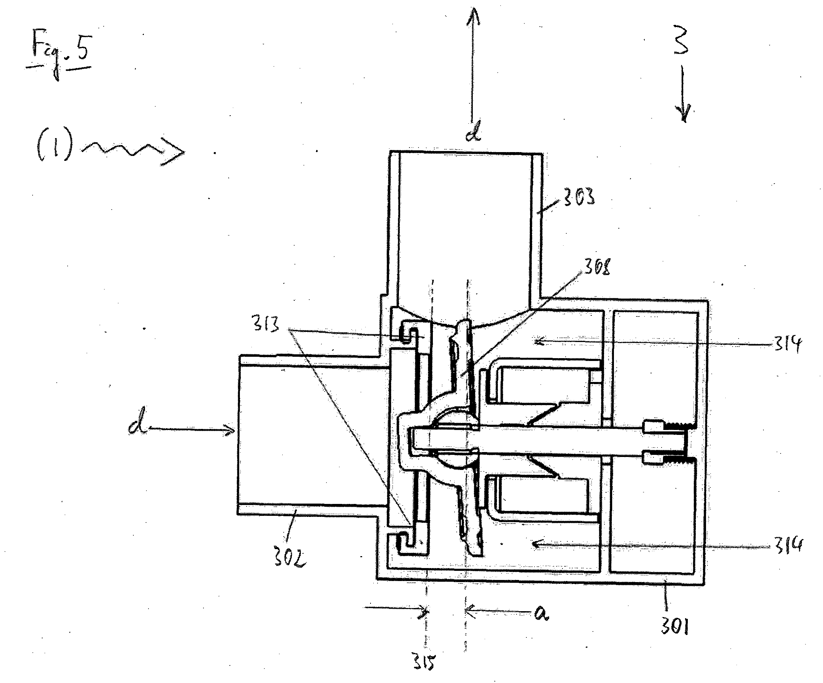

Die Fig. 5 zeigt einen Querschnitt durch das Magnetventil 3 des Beatmungsgerätes 1 aus Fig. 4 im geöffneten Zustand. Im geöffneten Zustand ist ein elektrischer Strom an dem Hubelektromagneten 304 geschaltet. Zur Verdeutlichung des mechanischen Spiels, welches dem Dichtteller 308 gegeben ist, ist hierfür der Dichtteller 308 zeichnerisch mit einer Seitenneigung dargestellt. Diese Darstellung entspricht einem fiktiven Zustand ohne Gasfluss. Beim Betrieb des beanspruchten Beatmungsgerätes 1 und geöffneten Zustand des Magnetventils 3 ist ein Gasfluss sowohl auf der dem Zufluss 302 zugewandten Seite des Dichttellers 308 als auch dahinter konstruktionsbedingt ermöglicht, indem hierfür um den Hubelektromagneten 304 und innerhalb des Ventilgehäuses 301 Freiräume 314 für einströmendes Gas gegeben sind. Beim Betrieb des beanspruchten Beatmungsgerätes 1 und geöffneten Zustand des Magnetventils 3 entspricht hierfür weiter das mathematische Produkt aus dem Umfang des Dichttellers und dem Abstand a zwischen dem Dichtteller 308 und dem durch die Flanschdichtung 313 ausgebildeten Rand 315 des Zulaufes 302 dem Strömungsquerschnitt S. Durch diese konstruktiven Merkmale ist ein Erhalt der Laminarströmung eines Gasflusses durch das Magnetventil 3 ohne Strömungsturbulenzen gewährleistet.

Das Ventil ist als Magnetventil 3 ausgebildet mit einem in dem Ventilgehäuse 301 fixierten Hubelektromagneten 304 und mit einem magnetisch bewegbaren Ventilkolben 305. Der Ventilkolben 305 weist einen Dichtteller 308 mit einer Dichtung auf, wobei der Dichtteller 308 den Zulauf 302 wirkt, also einen Gasfluss vom Elektrogebläse absperren kann. Der Ventilkolben 305 mit dem Dichtteller 308 wird durch eine Feder 306 gegen den Zulauf (302, 312) gepresst, sodass ein Gasfluss von dem oder zu dem Elektrogebläse unterbunden ist. Im geschlossenen Zustand des Magnetventils 3, ist der Hubelektromagnet 304 stromlos.

Wird der der Hubelektromagnet 304 bestromt, so wirkt eine einstellbare oder vorbestimmte Magnetkraft auf den magnetisch bewegbaren Ventilkolben 305. Der magnetisch bewegbare Ventilkolben 305 komprimiert dann die Feder 306 vorgebbar weit. In einem geöffneten Zustand ist die Magnetkraft die auf den magnetisch bewegbaren Ventilkolben 305 einwirkt größer als die Federkraft.

Im geschlossenen Zustand des Magnetventils 3, der Hubelektromagnet 304 ist stromlos ist, ist durch die Rückstellfeder 306 der Dichtteller 308 zusammen mit der Dichtkante 310 auf die Flanschdichtung 313 aufgepresst.

Der Zulauf 302 ist somit als Ventilsitz 312 ausgebildet, wobei der Dichtteller 308 gegenüber einer elastischen Flanschdichtung 313 auf dem Zulauf 302 angeordnet ist.

Die Rückstellfeder 306 ist somit in Richtung der Longitudinalachse l expandierbar, wobei mit dem elektrisch eingeschalteten Hubelektromagneten 304 eine durch den Ventilkolben 305 ausgeübte Kraft größer als die Rückstellkraft einstellbar ist.

Der Öffnungsquerschnitt des Ventils beträgt zumindest 180 mm² und bevorzugt rund 230mm².

Dieser Aufbau hat den Vorteil, dass im Falle eines Stromausfalls oder eines Fehlers, das Ventil automatisch schließt und für die geschlossen-Stellung keine Energie aufgewendet werden muss.

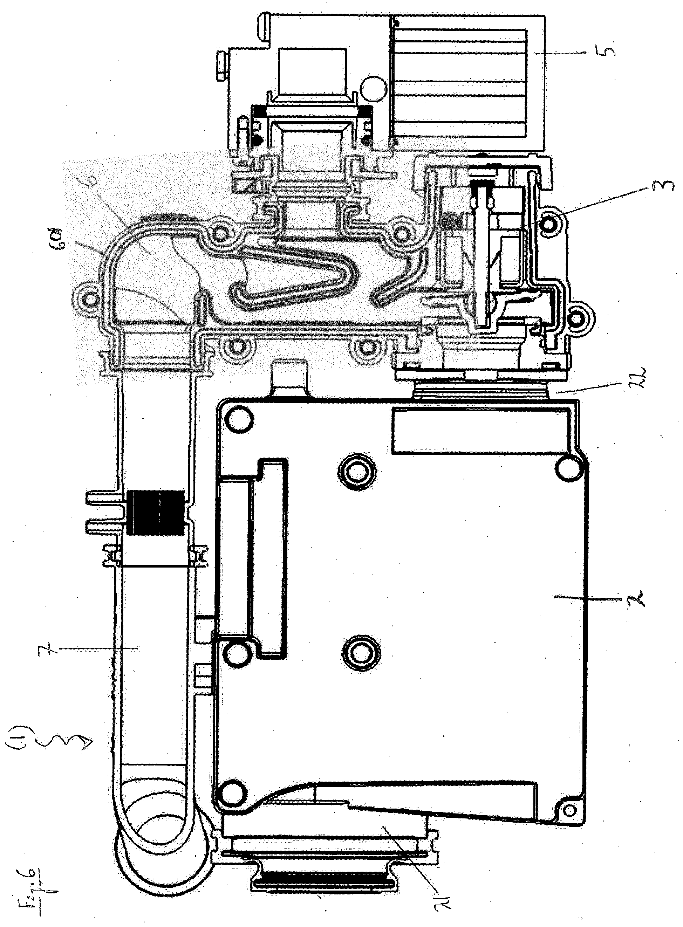

Die Fig. 6 zeigt eine teilgeschnittene Seitenansicht des Beatmungsgerätes 1, welches analog zu dem zweiten Ausführungsbeispiel zur Beatmung eines Patienten mit Atemgas ausgebildet ist, jedoch als drittes Ausführungsbeispiel eine Mischkammer 6 aufweist, in welcher das Ventil 3 integriert ist. Somit weist die Mischkammer ein Mischkammergehäuse 601 auf, welches in seiner Funktion auch dem Ventilgehäuse 301 entspricht. In der Fig 6. ist wieder als Ausschnitt des Beatmungsgerätes 1 die pneumatische Hauptleitung 4 gezeigt. Das Beatmungsgerät 1 in diesem dritten Ausführungsbeispiel zeichnet sich durch eine besonders kompakte Bauform aus.

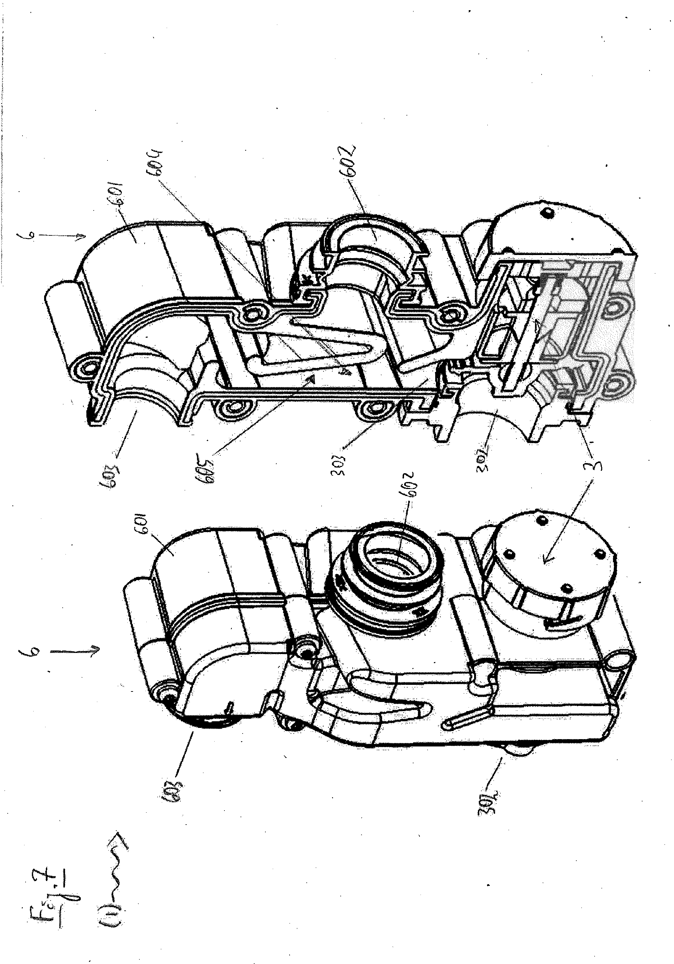

Die Fig. 7 zeigt eine Schrägansicht der Mischkammer 6 des dritten Ausführungsbeispiels, in welcher das Ventil 3 integriert ist, links in geschlossener Form, rechts im Querschnitt. Die Mischkammer 6 weist in ihrem ansonsten gasdicht gefertigten Mischkammergehäuse 601 als Öffnungen den Zulauf 302 des Ventils 3, einen Anschluss 602 für die Gasdosierungseinheit 5 und einen Anschluss 603 für die Zuleitung 7 auf. Das Mischkammergehäuse 601 ist durch Verschraubung und/oder formschlüssig gefertigt. Das Mischkammergehäuse 601 weist eine Innenkammer 604 mit einem Labyrinth 605 auf. In diesem Ausführungsbeispiel ist die Mischkammer 6 mit dem Labyrinth 605 einstückig z.B. aus ABS gefertigt. Der Auslauf 303 des Ventils 3 ist direkt in die Innenkammer 604 geführt, wobei der Auslauf 303 bezüglich der Flächengröße wieder den gleichen Strömungsquerschnitt S wie der Zulauf 302 aufweist und sich lediglich in der Geometrie unterscheidet. Ansonsten ist das Ventil 3 konstruktiv mit dem in Fig. 3 bis Fig. 5 gezeigten identisch. Auch in dieser Ausführungsform ist daher bei einem Gasfluss durch das Ventil 3 eine Laminarströmung gewährleistet. In der Fig. 7 ist das Ventil 3 geschlossen gezeigt.

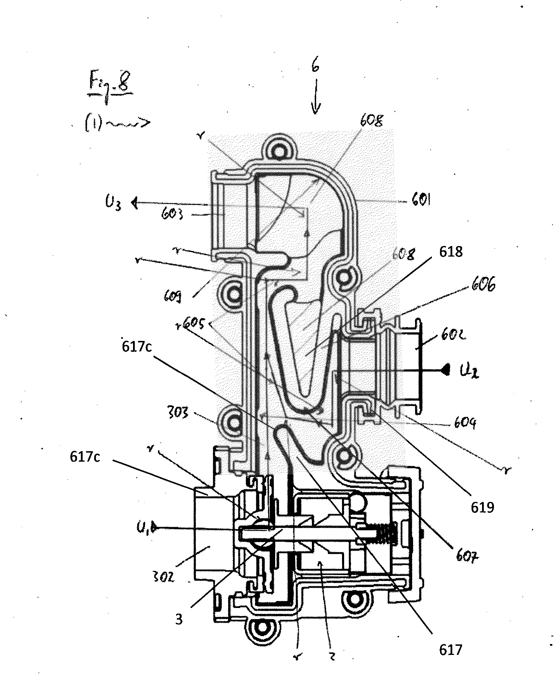

Die Fig. 8 zeigt einen Querschnitt der Seitenansicht der Mischkammer 6 des dritten Ausführungsbeispiels, in welcher das Ventil 3 integriert ist, unter Verdeutlichung von Umlenkungen r von Gasflüssen. Die Innenkammer 604 der Mischkammer 6 weist vor dem Anschluss 602 für die Gasdosierungseinheit 5 einen Umlenkkeil 606 mit einer Keilspitze 607 auf. Die Umlenkkeil 606 kann hierbei auch als Bestandteil des Labyrinths 605 aufgefasst werden. Der Umlenkkeil 606 bildet für den Strömungsweg eine Sackgasse 618 aus. Die Sackgasse 618 ist ein konstruktiver Auffangbereich für Resonanzen. Für die Schallwellen dient dieser Bereich der beispielsweise vollständig mit Dämmmaterial 608 gefüllt ist, als Resonanz-und Auffangbecken für Schallwellen, die darin "gefangen" werden und so den Schall dämmen.

Eine Füllung aus Dämmmaterial beispielsweise makroporösem Schaumstoff 608 ist beispielsweise sowohl in dem Umlenkkeil 606 und/oder auch auf der Oberfläche 609 der Innenkammer 604 eingebracht. In der Fig. 8 sind Fluss u₁ der geförderten Außenluft, der Fluss des eingespeisten Zusatzgases Sauerstoff u₂ und der Fluss des Atemgases u₃ durch jeweils verschiede zusammengesetzte Pfeile zeichnerisch verdeutlicht. In dem Labyrinth 605 weist der Fluss u₁ der geförderten Außenluft eine, der Fluss u₂ des eingespeisten Zusatzgases Sauerstoff zwei und der Fluss u₃ des Atemgases drei Umlenkungen r auf. Die Umlenkungen sorgen dafür, dass die Schallwellen zurück reflektiert werden und sich nicht ungehindert ausbreiten können. Der Fluss u₂ des eingespeisten Zusatzgases Sauerstoff wird zumindest zweifach bevorzugt dreifach umgelenkt; er wird zumindest einmal um mehr als 45° bevorzugt mehr als 70° und beispielsweise 90° umgelenkt und wird zumindest ein weiteres Mal um mehr als 45° bevorzugt mehr als 70° und beispielsweise 90° 90° umgelenkt und wird schließlich um mehr als 45° bevorzugt mehr als 70° und beispielsweise 100° umgelenkt.

Der Fluss u₁ der geförderten Außenluft wird zumindest einmal um mehr als 45° bevorzugt mehr als 70° und beispielsweise 90° umgelenkt.
der Fluss u₃ des Atemgases (02/Luft Gemisch) wird zumindest zweifach bevorzugt dreifach umgelenkt; er wird zumindest einmal um mehr als 45° bevorzugt mehr als 70° und beispielsweise 90° umgelenkt und wird zumindest ein weiteres Mal um mehr als 45° bevorzugt mehr als 70° und beispielsweise 90° umgelenkt und wird schließlich um mehr als 45° bevorzugt mehr als 70° und beispielsweise 90° oder 180° umgelenkt.

Hierbei ist ebenfalls der Fluss u₂ des eingespeisten Zusatzgases Sauerstoff um die Keilspitze 607 herumgeführt, wodurch gleichzeitig besonders vorteilhaft eine gute Durchmischung sichergestellt ist. Durch Umlenkungen r ist eine Reflexion von Schallwellen bei Betrieb des Elektrogebläses 2 bewirkt, was besonders vorteilhaft eine Schalldämpfung des betriebenen Beatmungsgerätes 1 realisiert.

Die Trennwand 617 ist benachbart zur Keilspitze 607 angeordnet. Beispielsweise bildet sich hier im durchflossenen Querschnitt eine Engstelle. Die Trennwand 617 trennt das Ventil vom Sauerstoffstrom u2 ab.

Wenn der Sauerstoffstrom im Vergleich zu dem der Umgebungsluft u1 sehr groß ist, soll mit der Trennwand eine negative Beeinflussung der Dichtfunktion des Ventils verhindert werden. Die Trennwand 617 weist eine Seite 617a auf, die dem Sauerstoffstrom u2 zugewandt ist und eine Seite 617b auf die dem Strom der Umgebungsluft u1 zugewandt ist. Mit ihrer Spitze 617c weist die Trennwand 617 in die Innenkammer 604, wobei sich der Sauerstoffstrom u2 und die Umgebungsluft u1 an der Spitze 617c der Trennwand treffen und mischen.

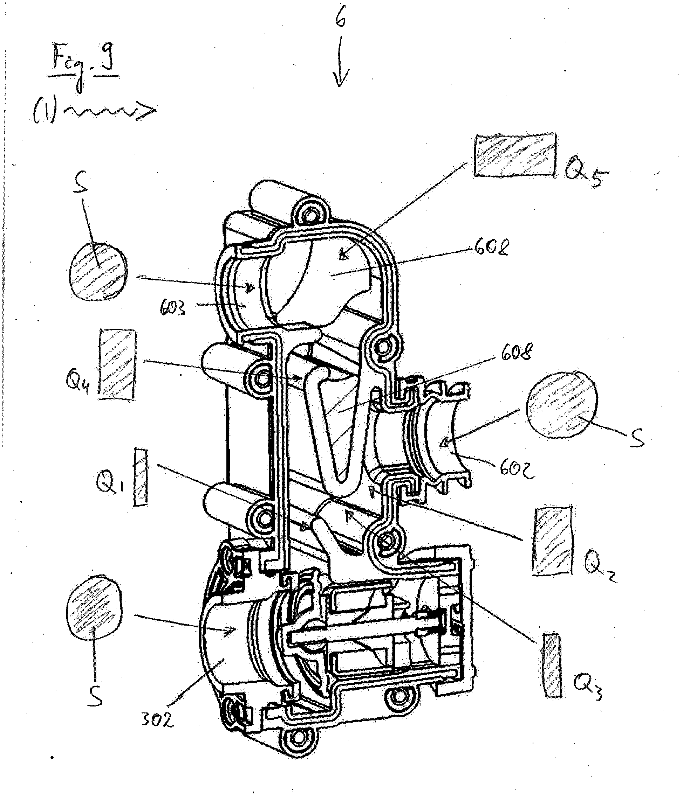

Die Fig. 9 zeigt eine geschnittene Schrägansicht der Mischkammer 6 des dritten Ausführungsbeispiels, in welcher das Ventil 3 integriert ist, unter Verdeutlichung von Änderungen eines jeweils durchflossenen Querschnittes Q₁, Q₂, Q₃, Q₄ und Q₅. In der Fig. 9 sind die jeweils von der geförderten Außenluft, von dem eingespeisten Sauerstoff durchflossenen Querschnitte Q₁, Q₂, Q₃, Q₄ und Q₅ als Flächen zur Verdeutlichung von deren Geometrien und im gleichen relativen Maßstab zur Verdeutlichung von deren Größenverhältnisse zueinander dargestellt, wobei die Positionen dieser einzelnen Querschnitte Q₁, Q₂, Q₃, Q₄ und Q₅ in der Mischkammer 6 durch Zuweisungspfeile in der Zeichnung der Mischkammer 6 dargelegt sind. In dem Labyrinth 605 weisen der Fluss u₁ der geförderten Außenluft, der Fluss u₂ des eingespeisten Zusatzgases Sauerstoff und der Fluss u₃ des Atemgases mindestens einmal eine Änderung der jeweils durchflossenen Querschnitte Q₁, Q₂, Q₃, Q₄ und Q₅ vertikal zu der Durchflussrichtung d auf, wodurch eine weitere Verbesserung der Schalldämpfung gegeben ist. Die durchflossenen Querschnitte Q₁, Q₂, Q₃, Q₄ und Q₅ entsprechen Strömungsquerschnitten. Ein gleichzeitiger Erhalt der Laminarströmung durch die Mischkammer 6 ist besonders vorteilhaft auch dadurch realisiert, dass sowohl der Strömungsquerschnitt sowohl des Anschlusses für die Gasdosierungseinheit 5 als auch des Anschlusses 603 für die Zuleitung 7 dem Strömungsquerschnitt S des Zulaufes 302 als auch der Auslaufes 303 des Ventils 3 bezüglich Flächengröße als auch Geometrie entsprechen. Somit ist Mischkammer 6 des dritten Ausführungsbeispiels sowohl bezüglich einer Baugrößenreduktion als auch bezüglich einer Schalldämmung unter gleichzeitigem erhalt der Laminarströmung optimiert.

Der Fluss u₁ der geförderten Außenluft tritt in den Zulauf 302 mit einem relativ großen Strömungsquerschnitt S ein, wobei der Zulauf beispielsweise einen runden Querschnitt aufweist. Der Fluss u₁ der geförderten Außenluft passiert das Ventil, wobei hier der durchflossene Querschnitt beispielsweise abnimmt, wobei jedoch bevorzugt im Bereich des Ventils 3 keine Engstelle besteht.

Der Fluss u₁ strömt weiter entlang der Trennwand 617 und trifft an der Spitze 617c der Trennwand 617 den Fluss u₂ des eingespeisten Zusatzgases. Nach der Mischstelle verringert sich der durchflossene Querschnitt bis zum Querschnitt Q4.

Der Fluss u₃ des gemischten Atemgases knickt dann dreifach um und verlässt die Mischkammer durch den Anschluss 603.

In einem vierten Ausführungsbeispiel ist das Ventil 3 als Proportionalventil ausgebildet. Dies gestattet besonders vorteilhaft eine präzise Einstellung und/oder Nachregelung von festgelegten Druck- und/oder Flusswerten des Narkosegases oder des Atemgases durch Einstellung des Abstandes a zwischen dem Dichtteller 308 und dem durch die Flanschdichtung 313 durch Regelung der Stromstärke am Hubelektromagneten 304. Ein Erhalt der Laminarströmung ist dann noch in einem ausreichenden Maße gegeben, wenn das mathematische Produkt aus dem Umfang des Dichttellers und dem Abstand a zwischen dem Dichtteller 308 und dem Rand 315 des Zulaufes 302 nicht mehr als 20 % vom Strömungsquerschnitt S des Zulaufes 302 abweicht.

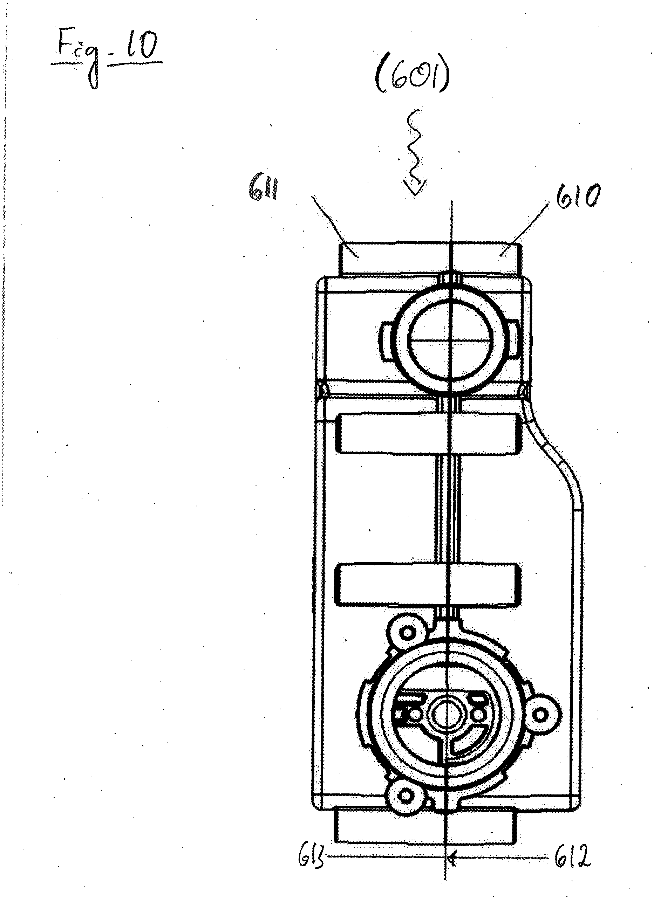

Die Fig. 10 zeigt eine Seitenansicht eines form- und kraftschlüssig gefertigten Bauteils 601, beispielsweise eines Mischkammergehäuses 601, aus einem Baustück 610 und einem Gegenstück 611. Das Baustück 610 weist eine Verbindungskante 612 und das Gegenstück 611 eine Verbindungskante 612 auf, welche jeweils zueinander formschlüssig sind. Bei diesem aus dem Baustück 610 und dem Gegenstück 611 gefertigten Bauteil 601 ist es zusätzlich konstruktiv gegeben, dass die Öffnungen für die Anschlüsse 602 und des Zulaufes 302 durch die Verbindungskanten 612, 613 geteilt sind. Das Baustück 610 und das Gegenstück 611 sind aus ABS gefertigt.

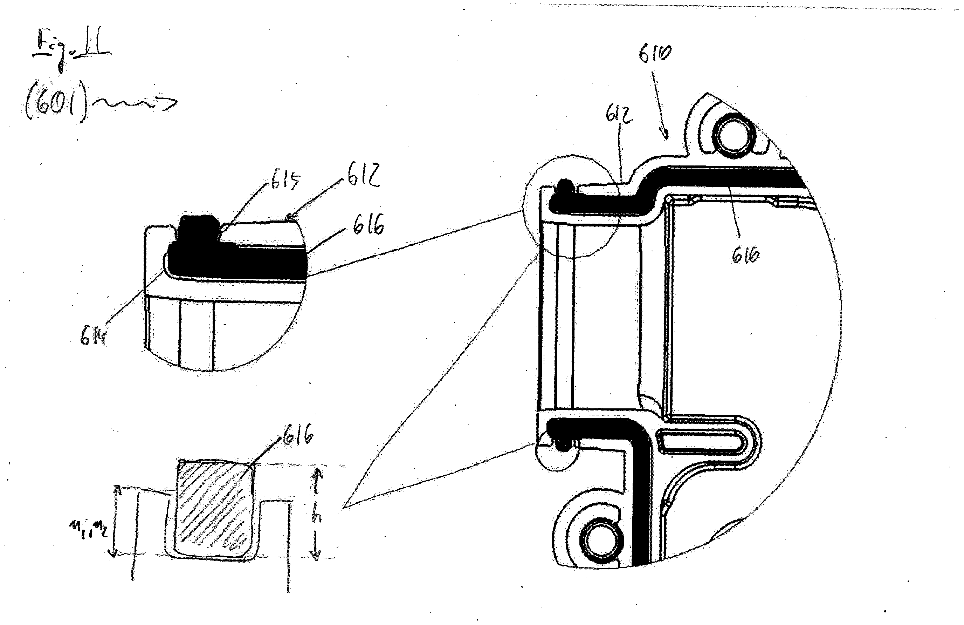

Die Fig. 11 zeigt eine Aufsicht auf eine Verbindungskante 612 des Baustückes 610 aus Fig. 10 unter Verdeutlichung der Verbindungskante 612 mit einer Nut 614, einer Quernut 615 und einer Flachdichtung 616. Hierbei zeigt die Fig. 11 rechts einen Ausschnitt aus der Verbindungskante 612 an der Stelle des Anschlusses 603 für die Zuleitung 7, links oben und links unten jeweils eine vergrößerte Ansicht auf die Nut 614 mit der Quernut 615 der Flachdichtung 616.

An den in der Fig. 11 gezeigten Stelle des Baustückes 610 ist in die Verbindungskante 612 eine Nut 614 parallel zu der Länge der Verbindungskante 612 und jeweils auf der gegenüberliegenden Seite des Anschlusses 603 eine in die Nut 614 vertikal mündende und die betreffende Verbindungskante 612 durchbrechende Quernut 615 eingelassen, wobei eine sowohl in der Nut 614 als auch in der jeweiligen Quernut 615 verlaufende einstückige elastische und komprimierbare Flachdichtung 616 eingebracht ist. Die Flachdichtung 616 ist aus Silikon gefertigt. Die Flachdichtung 616 ist formschlüssig sowohl zu der Nut 614 als auch zu jeder Quernut 615. Der Flachdichtung 616 weist eine größere Dichtungshöhe h als die Tiefe n₁ der Nut 614 als auch die Tiefe n₂ der Quernut 615 auf. Die Dichtungshöhe beträgt bei diesem Ausführungsbeispiel das 1,2-fache der Tiefe n₁ der Nut 614, wobei die Tiefe n₁ der Nut 614 gleich der Tiefe n₂ der Quernut 615. Die Flachdichtung 616 ist über die Quernut 615 durch die Verbindungkante 612 um das 1,2-fache der Tiefe n₂ der Quernut 615 entsprechend hinausragend gestaltet. Durch Kompression der Flachdichtung 616 in der Quernut 615 durch Form- und Kraftschluss zu dem Bauteil 601 ist an der betreffenden Stelle der Verbindungskante 612 die Abdichtung verstärkt.

### Bezugszeichenliste

- 1: Beatmungsgerät
- 2: Elektrogebläse
- 21: Ansaugeingang
- 22: Gebläseausgang
- 3: (Magnet)Ventil
- 301: Ventilgehäuse
- 302: Zulauf
- 303: Auslauf
- 304: Hubelektromagneten
- 305: Ventilkolben
- 306: Rückstellfeder
- 307: Kugel
- 308: Dichtteller
- 309: Kugelpfanne
- 310: Dichtkante
- 311: Kugelgelenk
- 312: Ventilsitz
- 313: Flanschdichtung
- 314: Freiräume
- 315: Rand
- 4: pneumatische Hauptleitung
- 5: Gasdosierungseinheit
- 51: Einspeisungseingang für Außenluft
- 52: Einspeisungseingang für Sauerstoff
- 53: Einspeisungseingang für Narkosegas (Lachgas)
- 6: Mischkammer
- 601: Mischkammergehäuse
- 620: Anschluss für Atemgasquelle 2
- 602: Anschluss für Gasdosierungseinheit 5
- 603: Anschluss für Zuleitung 7
- 604: Innenkammer
- 605: Labyrinth
- 606: Umlenkkeil
- 607: Keilspitze
- 608: Dämmmaterial (makroporöser Schaumstoff)
- 609: Oberfläche
- 610: Baustück
- 611: Gegenstück
- 612: Verbindungskante des Baustückes 610
- 613: Verbindungskante des Gegenstückes 611
- 614: Nut
- 615: Quernut
- 616: Flachdichtung
- 617: Trennwand
- 618: Sackgassse
- 7: Zuleitung
- 8: Steuerung
- a: Abstand
- d: Durchflussrichtung
- h: Dichtungshöhe
- l: Longitudinalachse
- n₁: Tiefe der Nut 614
- n₂: Tiefe der Quernut 615
- Q₁: Querschnitt
- Q₂: Querschnitt
- Q₃: Querschnitt
- Q₄: Querschnitt
- Q₅: Querschnitt
- r: Umlenkung
- S: Strömungsquerschnitt
- u₁: Fluss der geförderten Außenluft
- u₂: Fluss des eingespeisten Zusatzgases
- u₃: Fluss des Atemgases

## Patentansprüche

1. Beatmungsgerät (1) mit einer elektronischen Steuervorrichtung (8) sowie einer pneumatischen Hauptleitung (4) in der pneumatisch verbunden angeordnet sind; eine Atemgasquelle (2), die als Elektrogebläse (2) mit einem Ansaugeingang (21) zur Bereitstellung von Außenluft und einem Gebläseausgang (22; ausgebildet ist, zumindest einem Ventil (3), einer Mischkammer (6) und, einer Gasdosierungseinheit (5) und einer Zuleitung (7) wobei die Gasdosierungseinheit (5) für eine einstellbare pneumatische Einspeisung von einem Sauerstoff (52) enthaltenden Zusatzgas zu oder anstelle der geförderten Außenluft in die Mischkammer (6) ausgebildet ist und dass die Atemgasquelle (2) eingerichtet ist Atemgas zu der Zuleitung (7) zu fördern und dass die Mischkammer (6) eingerichtet ist Atemgas bereitzustellen, wobei die Zuleitung (7) zur Versorgung des Patienten mit Atemgas eingerichtet ist, wobei das Ventil (3) eingerichtet ist, einen Atemgasstrom zum Patienten zumindest zeitweise zu reduzieren oder zu unterbrechen, wobei das Atemgas Außenluft und/oder Sauerstoff aufweist, wobei das Ventil als Teil der Mischkammer (6) ausgebildet ist oder in einem gemeinsamen Gehäuse der Mischkammer angeordnet ist, wobei das Ventil in der pneumatischen Hauptleitung (4) in Durchflussrichtung (d) nach dem Gebläseausgang (22) angeordnet ist und in Durchflussrichtung (d) vor der Gasdosierungseinheit (5), wobei das Ventil (3) mit der Mischkammer (6) und die Mischkammer (6) sowohl mit der Gasdosierungseinheit (5) als auch mit der Zuleitung (7) pneumatisch verbunden ist, wobei die Gasdosierungseinheit (5) in Durchflussrichtung (d) vor der Zuleitung (7) angeordnet ist,
wobei mit der Steuervorrichtung der Zusatzgasanteil, der Beatmungsdruck und ein Beatmungsfluss des Atemgases einstellbar ist und **dadurch gekennzeichnet, dass** die Steuervorrichtung zusätzlich zur Absperrung des Ventils unter gleichzeitiger Öffnung der Gasdosierungseinheit sowie die Gasdosierungseinheit selbst zur Bereitstellung von Atemgas bei Ausfall des Elektrogebläses und/oder der Stromversorgung und/oder bei Ausfall des Prozessors und/oder bei Absturz der Software ausgebildet ist.

2. Beatmungsgerät (1) nach Anspruch 1 wobei die Zuleitung (7) zur Versorgung des Patienten mit einem Atemgas bestehend aus der geförderten Außenluft oder einem Gasgemisch aus der Außenluft und dem Zusatzgas oder aus dem Zusatzgas allein eingerichtet ist.

3. Beatmungsgerät (1) nach zumindest einem der vorhergehenden Ansprüche **dadurch gekennzeichnet, dass** die Mischkammer (6) einen Anschluss (602) für die Gasdosierungseinheit (5), einen Anschluss (603) für die Zuleitung (7) und einen Anschluss 620 für die Atemgasquelle (2) aufweist.

4. Beatmungsgerät (1) nach zumindest einem der vorhergehenden Ansprüche **dadurch gekennzeichnet, dass** das Ventil (3), aufweisend einen Zulauf (302) und einen Auslauf (303) in einem Ventilgehäuse (301), über den Auslauf (303) mit dem Ansaugeingang (21) oder über den Zulauf (302) mit dem Gebläseausqanq (22) pneumatisch verbunden ist, wobei das Elektroqebläse (2) und das Ventil (3) mit der Steuervorrichtung (8) in mindestens einem gemeinsamen Regelkreis elektronisch geregelt sind und die Steuervorrichtung (8) selbst zumindest teilweise über einen Algorithmus in Form von Software mit einem Prozessor elektronisch regelbar und/oder automatisch geregelt ist, wobei in oder an der pneumatischen Hauptleitung (4) und/oder in weiteren pneumatischen Zweig- und/oder Nebenleitungen und/oder Rückführungen funktionelle Baugruppen (5, 6, 7) und optional Mess- und/oder Regeleinstrumente pneumatisch geschaltet sind, wobei die funktionellen Baugruppen (5, 6, 7) mittels der Steuervorrichtung (8) elektronisch geregelt und die Messund/oder Regeleinstrumente ebenfalls optionale Baugruppen der Steuervorrichtung (8) sind.

5. Beatmungsgerät (1) nach zumindest einem der vorhergehenden Ansprüche **dadurch gekennzeichnet, dass** das Ventil als Magnetventil 3 ausgebildet ist mit einem in einem Ventilgehäuse 301 fixierten Elektromagneten 304 und mit einem magnetisch bewegbaren Ventilkolben 305, wobei der Ventilkolben 305 einen Dichtteller 308 mit einer Dichtung aufweist, wobei der Dichtteller 308 auf einen Zulauf 302 wirkt und wobei der Ventilkolben 305 mit dem Dichtteller 308 durch eine Feder 306 gegen den Zulauf (302, 312) gepresst wird, sodass ein Gasfluss von dem oder zu dem Elektrogebläse unterbunden ist.

6. Beatmungsgerät (1) nach zumindest Anspruch 5 **dadurch gekennzeichnet, dass** der Elektromagnet (304) im geschlossenen Zustand des Magnetventils (3), stromlos ist.

7. Beatmungsgerät (1) nach zumindest Anspruch 5 oder 6 **dadurch gekennzeichnet, dass** wenn der Elektromagnet (304) bestromt wird, eine einstellbare oder vorbestimmte Magnetkraft auf den magnetisch bewegbaren Ventilkolben (305) wirkt, wobei der magnetisch bewegbare Ventilkolben (305) die Feder (306) vorgebbar weit komprimiert, wobei in einem geöffneten Zustand die Magnetkraft, die auf den magnetisch bewegbaren Ventilkolben (305) einwirkt, größer ist als die Federkraft.

8. Beatmungsgerät (1) nach zumindest einem der vorhergehenden Ansprüche **dadurch gekennzeichnet, dass** die Steuervorrichtung (8) zumindest einen Prozessor (oder Computer) aufweist oder mehrere Prozessoren aufweist um zumindest das Elektrogebläse, das Ventil und Mess- und/oder Regeleinstrumente zu steuern.

9. Beatmungsgerät (1) nach zumindest einem der vorhergehenden Ansprüche wobei mit der Steuervorrichtung (8) der Zusatzgasanteil, der Beatmungsdruck und ein Beatmungsfluss des Atemgases einstellbar ist.

10. Beatmungsgerät (1) nach zumindest einem der vorhergehenden Ansprüche **dadurch gekennzeichnet, dass** das Ventil (3) direktgesteuert ist, hierfür einen Hubelektromagneten (304) mit einem Ventilkolben (305) aufweist und durch Stromregelung am Hubmagneten (305) betreibbar ist, wobei vorderseitig und vertikal auf dem Ventilkolben (305) ein Dichtteller (308) angebracht ist, wobei der Zulauf (302) als Ventilsitz (312) ausgebildet ist, wobei dem Dichtteller (308) ein mechanisches Spiel mit drei Freiheitsgraden gegeben ist, wobei der Zulauf (302) als auch der Auslauf (303) jeweils einen bezüglich Flächengröße gleichen Strömungsquerschnitt (S) aufweisen, wobei bei geöffnetem Zustand des Ventils (3) ein Gasfluss sowohl auf der dem Zufluss (302) zugewandten Seite des Dichttellers (308) als auch dahinter konstruktionsbedingt ermöglicht ist und das mathematische Produkt aus dem Umfang des Dichttellers (308) und dem Abstand (a) zwischen dem Dichtteller (308) und dem Rand (315) des Zulaufes (302) dem Strömungsquerschnitt (S) mit einer Abweichung von nicht mehr als 20 % entspricht.

11. Beatmungsgerät (1) nach zumindest einem der vorhergehenden Ansprüche **dadurch gekennzeichnet, dass** die Mischkammer (6) als Ventilgehäuse (301) ausgebildet ist, wobei der Zulauf (302) des Ventils (3) als Zulauf (302) zu der Mischkammer (6) ausgebildet ist und der Auslauf (303) in eine Innenkammer (604) der Mischkammer (6) pneumatisch geführt ist, wobei die Innenkammer (604) sowohl mit der Gasdosierungseinheit (5) als auch mit der Zuleitung (7) pneumatisch verbunden ist und wobei die Innenkammer (604) ein Labyrinth (605) aufweist.

12. Beatmungsgerät (1) nach zumindest Anspruch 11 **dadurch gekennzeichnet, dass** in dem Labyrinth (605) der Fluss (u₁) der geförderten Außenluft und/oder der Fluss des eingespeisten Zusatzgases (u₂) und/oder der Fluss des Atemgases (u₃) jeweils mindestens einmal eine Umlenkung (r) aufweist oder aufweisen oder/und der Fluss (u₁) der geförderten Außenluft und/oder der Fluss (u₂)des eingespeisten Zusatzgases und/oder der Fluss (u₃) des Atemgases jeweils mindestens einmal eine Änderung des durchflossenen Querschnittes (Q₁, Q₂, Q₃, Q₄, Q₅) vertikal zu der Durchflussrichtung (d) aufweist oder aufweisen.

13. Beatmungsgerät (1) nach zumindest einem der vorhergehenden Ansprüche **dadurch gekennzeichnet, dass** die Mischkammer (6) vor der Gasdosierungseinheit (5) einen Umlenkkeil (606) mit einer Keilspitze (607) aufweist, wobei der Fluss (u₂)des eingespeisten Zusatzgases um die Keilspitze (607) herumgeführt ist und wobei der Umlenkkeil (606) hohlförmig ausgebildet ist und/oder eine Füllung aus Dämmmaterial (608) aufweist.

14. Beatmungsgerät (1) nach zumindest Anspruch 11 oder 12 **dadurch gekennzeichnet, dass** in dem Labyrinth (605) eine Oberfläche (609) gegeben ist, welche zumindest teilweise mit Dämmmaterial (608) ausgekleidet ist und/oder wobei das Dämmmaterial (608) optional antimikrobielle Eigenschaften aufweist.

## Claims

1. A ventilator (1) having an electronic control device (8) as well as a pneumatic main line (4) in which the following are arranged, connected pneumatically; a respiratory gas source (2) which is configured as an electric fan (2) having a suction input (21) for making available respiratory air and a fan output (22), at least one valve (3), a mixer chamber (6) and a gas-dosing unit (5) and a supply line (7), wherein the gas-dosing unit (5) is configured for adjustable pneumatic feeding of an auxiliary gas containing oxygen (52) in addition to or instead of the delivered external air into the mixer chamber (6) and the respiratory gas source (2) is adapted to deliver respiratory gas to the supply line (7) and the mixer chamber (6) is adapted to make available respiratory gas, wherein the supply line (7) is adapted to supply the patient with respiratory gas, wherein the valve (3) is adapted to at least temporarily reduce or interrupt a stream of respiratory gas to the patient, wherein the respiratory gas has external air and/or oxygen, wherein the valve is configured as part of the mixer chamber (6) or is arranged in a common housing of the mixer chamber, wherein the valve in the pneumatic main line (4) is arranged downstream from the fan output (22) in a direction of flow (d) and upstream from the gas-dosing unit (5) in a direction of flow (d), wherein the valve (3) is connected pneumatically to the mixer chamber (6) and the mixer chamber (6) is connected pneumatically both to the gas-dosing apparatus (5) and to the supply line (7), wherein the gas-dosing unit (5) is arranged upstream from the supply line (7) in a direction of flow (d), wherein the auxiliary gas fraction, the ventilation pressure and a ventilation flow of the respiratory gas can be adjusted with the control device, and **characterized in that** the control device is additionally configured to shut off the valve with simultaneous opening of the gas-dosing unit, and the gas-dosing unit itself is configured to make available respiratory gas in the event of a failure of the electric fan and/or of the power supply and/or in the event of a failure of the processor and/or in the event of the software crashing.

2. The ventilator (1) according to Claim 1, wherein the supply line (7) is adapted to supply the patient with a respiratory gas consisting of the delivered external air or a gas mixture of the external air and the auxiliary gas or the auxiliary gas alone.

3. The ventilator (1) according to at least one of the preceding claims, **characterized in that** the mixer chamber (6) has a port (602) for the gas-dosing unit (5), a port (603) for the supply line (7), and a port 620 for the respiratory gas source (2).

4. The ventilator (1) according to at least one of the preceding claims, **characterized in that** the valve (3), having an inlet (302) and an outlet (303) in a valve housing (301), is connected pneumatically via the outlet (303) to the suction input (21) or via the inlet (302) to the fan output (22), wherein the electric fan (2) and the valve (3) are electronically regulated with the control device (8) in at least one common control circuit, and the control device (8) itself can be electronically regulated and/or automatically regulated at least partially with a processor via an algorithm in the form of software, wherein functional assemblies (5, 6, 7) and optionally measuring and/or regulating instruments are connected pneumatically in or on the pneumatic main line (4) and/or in further pneumatic branch lines and/or secondary lines and/or return lines, wherein the functional assemblies (5, 6, 7) are electronically regulated by means of the control device (8), and the measuring and/or regulating instruments are likewise optional assemblies of the control device (8).

5. The ventilator (1) according to at least one of the preceding claims, **characterized in that** the valve is configured as a solenoid valve 3 with an electromagnet 304 fixed in a valve housing 301 and with a magnetically movable valve piston 305, wherein the valve piston 305 has a sealing plate 308 with a seal, wherein the sealing plate 308 acts on an inlet 302, and wherein the valve piston 305 is pressed with the sealing plate 308 against the inlet (302, 312) by a spring 306, such that a gas flow from or to the electric fan is suppressed.

6. The ventilator (1) according to at least Claim 5, **characterized in that** the electromagnet (304), in the closed state of the solenoid valve (3), is currentless.

7. The ventilator (1) according to at least Claim 5 or 6, **characterized in that** when current flows through the electromagnet (304), an adjustable or predetermined magnetic force acts on the magnetically movable valve piston (305), wherein the magnetically movable valve piston (305) compresses the spring (306) to a predefinable extent, wherein, in an opened state, the magnetic force acting on the magnetically movable valve piston (305) is greater than the spring force.

8. The ventilator (1) according to at least one of the preceding claims, **characterized in that** the control device (8) has at least one processor (or computer) or has several processors, in order to control at least the electric fan, the valve and measuring and/or regulating instruments.

9. The ventilator (1) according to at least one of the preceding claims, wherein the auxiliary gas fraction, the ventilation pressure and a ventilation flow of the respiratory gas can be adjusted with the control device (8) .

10. The ventilator (1) according to at least one of the preceding claims, **characterized in that** the valve (3) is directly controlled, for which purpose it has a lifting electromagnet (304) with a valve piston (305) and can be operated by current regulation at the lifting magnet (305), wherein a sealing plate (308) is mounted vertically on a front of the valve piston (305), wherein the inlet (302) is configured as a valve seat (312), wherein the sealing plate (308) is given mechanical play with three degrees of freedom, wherein the inlet (302) and also the outlet (303) each have an identical cross section of flow (S) with respect to surface area, wherein, in an opened state of the valve (3), a gas flow is made possible, due to the construction, both on the side of the sealing plate (308) facing toward the inlet (302) and also to the rear thereof, and the mathematical product of the circumference of the sealing plate (308) and the distance (a) between the sealing plate (308) and the edge (315) of the inlet (302) corresponds to the cross section of flow (S) with a deviation of not more than 20%.

11. The ventilator (1) according to at least one of the preceding claims, **characterized in that** the mixer chamber (6) is configured as a valve housing (301), wherein the inlet (302) of the valve (3) is configured as an inlet (302) to the mixer chamber (6), and the outlet (303) is guided pneumatically into an inner chamber (604) of the mixer chamber (6), wherein the inner chamber (604) is connected pneumatically both to the gas-dosing unit (5) and to the supply line (7), and wherein the inner chamber (604) has a labyrinth (605).

12. The ventilator (1) according to at least Claim 11, **characterized in that,** in the labyrinth (605), the flow (u₁) of the delivered external air and/or the flow of the fed-in auxiliary gas (u₂)and/or the flow of the respiratory gas (u₃) in each case has or have at least once a deflection (r), and/or the flow (u₁) of the delivered external air and/or the flow (u₂) of the fed-in auxiliary gas and/or the flow (u₃) of the respiratory gas in each case has or have at least once a change of the flowedthrough cross section (Q₁, Q₂, Q₃, Q₄, Qs) vertically with respect to the direction of flow (d).

13. The ventilator (1) according to at least one of the preceding claims, **characterized in that** the mixer chamber (6) upstream from the gas-dosing unit (5) has a deflection wedge (606) with a wedge tip (607), wherein the flow (u₂) of the fed-in auxiliary gas is routed around the wedge tip (607), and wherein the deflection wedge (606) is configured with a hollow shape and/or has a filler composed of insulating material (608).

14. The ventilator (1) according to at least Claim 11 or 12, **characterized in that,** in the labyrinth (605), a surface (609) is provided which is at least partially lined with insulating material (608) and/or wherein the insulating material (608) optionally has antimicrobial properties.

## Revendications

1. Appareil respiratoire (1) pourvu d'un dispositif de commande électronique (8) ainsi que d'une conduite principale pneumatique (4) dans laquelle sont disposés les éléments suivants, reliés de façon pneumatique ; une source de gaz respiratoire (2) conçue comme un souffleur électrique (2) pourvu d'une entrée d'aspiration (21) destinée à fournir de l'air respirable et d'une sortie de souffleur (22), au moins une soupape (3), une chambre de mélange (6) et une unité de dosage de gaz (5) et une conduite d'arrivée (7), dans lequel l'unité de dosage de gaz (5) est conçue pour introduire du gaz d'apport contenant de l'oxygène (52) en complément de l'air extérieur alimenté ou à la place de celui-ci de manière pneumatiquement réglable dans la chambre de mélange (6), et la source de gaz respiratoire (2) est configurée pour transporter du gaz respiratoire vers la conduite d'arrivée (7) et la chambre de mélange (6) est configurée pour fournir du gaz respiratoire, dans lequel la conduite d'arrivée (7) est configurée pour fournir du gaz respiratoire au patient, dans lequel la soupape (3) est configurée pour réduire ou interrompre au moins temporairement un flux de gaz respiratoire vers le patient, dans lequel le gaz respiratoire présente de l'air extérieur et/ou de l'oxygène, dans lequel la soupape est conçue comme une partie de la chambre de mélange (6) ou disposée dans un boîtier commun de la chambre de mélange, dans lequel la soupape est disposée dans la conduite principale pneumatique (4) en aval de la sortie de souffleur (22) dans la direction d'écoulement (d) et en amont de l'unité de dosage de gaz (5) dans la direction d'écoulement (d), dans lequel la soupape (3) est reliée pneumatiquement à la chambre de mélange (6) et la chambre de mélange (6) est reliée pneumatiquement aussi bien à l'unité de dosage de gaz (5) qu'à la conduite d'arrivée (7), dans lequel l'unité de dosage de gaz (5) est disposée en amont de la conduite d'arrivée (7) dans la direction d'écoulement (d), dans lequel le dispositif de commande permet de régler la part de gaz d'apport, la pression respiratoire et un flux respiratoire du gaz respiratoire et **caractérisé en ce que** le dispositif de commande est en outre conçu pour bloquer la soupape en ouvrant simultanément l'unité de dosage de gaz, et l'unité de dosage de gaz elle-même est conçue pour fournir du gaz respiratoire en cas de panne du souffleur électrique et/ou de l'alimentation électrique et/ou en cas de panne du processeur et/ou de plantage du logiciel.

2. Appareil respiratoire (1) selon la revendication 1, dans lequel la conduite d'arrivée (7) est configurée pour fournir au patient un gaz respiratoire constitué d'air extérieur alimenté ou d'un mélange gazeux composé de l'air extérieur et d'un gaz d'apport ou du gaz d'apport seul

3. Appareil respiratoire (1) selon l'une au moins des revendications précédentes, **caractérisé en ce que** la chambre de mélange (6) présente un raccord (602) pour l'unité de dosage de gaz (5), un raccord (603) pour la conduite d'arrivée (7) et un raccord 620 pour la source de gaz respiratoire (2).

4. Appareil respiratoire (1) selon l'une au moins des revendications précédentes, **caractérisé en ce que** la soupape (3), présentant une amenée (302) et une évacuation (303) dans un boîtier de soupape (301), est reliée pneumatiquement à l'entrée d'aspiration (21) par le biais de l'évacuation (303) ou à la sortie de souffleur (22) par le biais de l'amenée (302), dans lequel le souffleur électrique (2) et la soupape (3) sont réglés électroniquement à l'aide du dispositif de commande (8) dans au moins un circuit de réglage commun et le dispositif de commande (8) lui-même peut être réglé électroniquement ou est réglé automatiquement au moins partiellement par le biais d'un algorithme sous la forme de logiciel à l'aide d'un processeur , dans lequel des modules fonctionnels (5, 6, 7) et facultativement des instruments de mesure et/ou de réglage sont montés pneumatiquement dans ou sur la conduite principale pneumatique (4) et/ou dans d'autres conduites secondaires ou de raccordement pneumatiques et/ou conduites de retour, dans lequel les modules fonctionnels (5, 6, 7) sont réglés électroniquement à l'aide du dispositif de commande (8) et les instruments de mesure et/ou de réglage sont également des modules facultatifs du dispositif de commande (8).

5. Appareil respiratoire (1) selon l'une au moins des revendications précédentes, **caractérisé en ce que** la soupape est conçue comme une soupape magnétique 3 avec un électroaimant 304 fixé dans un boîtier de soupape 301 et avec un piston de soupape 305 déplaçable magnétiquement, dans lequel le piston de soupape 305 présente une tête d'étanchéité 308 avec un joint d'étanchéité, dans lequel la tête d'étanchéité 308 agit sur une amenée 302 et dans lequel le piston de soupape 305 est pressé avec la tête d'étanchéité 308 contre l'amenée (302, 312) par un ressort 306, de manière à empêcher un flux de gaz de s'écouler à partir du souffleur électronique ou vers celui-ci.

6. Appareil respiratoire (1) au moins selon la revendication 5, **caractérisé en ce que** l'électroaimant (304) est exempt de courant dans l'état fermé de la soupape magnétique (3).

7. Appareil respiratoire (1) au moins selon la revendication 5 ou 6, **caractérisé en ce que** lorsque l'électroaimant (304) est alimenté en courant, une force magnétique réglable ou prédéfinie agit sur le piston de soupape (305) déplaçable magnétiquement, dans lequel le piston de soupape (305) déplaçable magnétiquement comprime le ressort (306) à un degré prédéfinissable, dans lequel la force magnétique agissant sur le piston de soupape (305) déplaçable magnétiquement est supérieure à la force de ressort dans un état ouvert.

8. Appareil respiratoire (1) selon l'une au moins des revendications précédentes, **caractérisé en ce que** le dispositif de commande (8) présente au moins un processeur (ou un ordinateur) ou présente plusieurs processeurs, afin de commander au moins le souffleur électrique, la soupape et des instruments de mesure et/ou de réglage.

9. Appareil respiratoire (1) selon l'une au moins des revendications précédentes, dans lequel le dispositif de commande (8) permet de régler la part de gaz d'apport, la pression respiratoire et un flux respiratoire du gaz respiratoire.

10. Appareil respiratoire (1) selon l'une au moins des revendications précédentes, **caractérisé en ce que** la soupape (3) est commandée directement, présente à cet effet un électroaimant de levage (304) avec un piston de soupape (305) et peut être actionnée par un réglage de courant au niveau de l'aimant de levage (305), dans lequel une tête d'étanchéité (308) est fixée du côté avant et verticalement sur le piston de soupape (305), dans lequel l'amenée (302) est conçue comme un siège de soupape (312), dans lequel la tête d'étanchéité (308) est dotée d'un jeu mécanique avec trois degrés de liberté, dans lequel l'amenée (302) ainsi que l'évacuation (303) présentent respectivement une section transversale d'écoulement (S) identique dans la superficie, dans lequel, dans l'état ouvert de la soupape (3), un flux de gaz peut s'écouler aussi bien sur le côté de la tête d'étanchéité (308) tourné vers l'amenée (302) qu'à l'arrière de celle-ci, en fonction de la construction, et le produit mathématique de la circonférence de la tête d'étanchéité (308) et de la distance (a) entre la tête d'étanchéité (308) et le bord (315) de l'amenée (302) correspond à la section transversale d'écoulement (S) avec un écart ne dépassant pas 20%.

11. Appareil respiratoire (1) selon l'une au moins des revendications précédentes, **caractérisé en ce que** la chambre de mélange (6) est conçue comme un boîtier de soupape (301), dans lequel l'amenée (302) de la soupape (3) est conçue comme l'amenée (302) vers la chambre de mélange (6) et l'évacuation (303) est guidée pneumatiquement dans une chambre intérieure (604) de la chambre de mélange (6), dans lequel la chambre intérieure (604) est reliée pneumatiquement aussi bien à l'unité de dosage de gaz (5) qu'à la conduite d'arrivée (7) et dans lequel la chambre intérieure (604) présente un labyrinthe (605).

12. Appareil respiratoire (1) au moins selon la revendication 11, **caractérisé en ce que** dans le labyrinthe (605), le flux (u₁) de l'air extérieur alimenté et/ou le flux du gaz d'apport introduit (u₂) et/ou le flux du gaz respiratoire (u₃) présente ou présentent respectivement au moins une fois une déviation (r) ou/et le flux (u₁) de l'air extérieur alimenté et/ou le flux (u₂) du gaz d'apport introduit et/ou le flux (u₃) du gaz respiratoire présente ou présentent respectivement au moins une fois une modification de la section transversale de passage (Q₁, Q₂, Q₃, Q₄, Qs) verticalement à la direction d'écoulement (d) .

13. Appareil respiratoire (1) selon l'une au moins des revendications précédentes, **caractérisé en ce que** la chambre de mélange (6) présente une clavette de déviation (606) dotée d'une pointe de clavette (607) en amont de l'unité de dosage de gaz (5), dans lequel le flux (u₂)du gaz d'apport introduit est guidé autour de la pointe de clavette (607) et dans lequel la clavette de déviation (606) est conçue avec une forme creuse et/ou présente une garniture constituée de matériau isolant (608).

14. Appareil respiratoire (1) au moins selon la revendication 11 ou 12, **caractérisé en ce que** dans le labyrinthe (605), il est prévu une surface (609) au moins partiellement revêtue de matériau isolant (608) et/ou dans lequel le matériau isolant (608) présente facultativement des propriétés antimicrobiennes.
